(19) Europäisches Patentamt
European Patent Office
Office européen des brevets

(11) **EP 3 692 962 A1**

(12) **EUROPEAN PATENT APPLICATION**
published in accordance with Art. 153(4) EPC

(43) Date of publication:
**12.08.2020 Bulletin 2020/33**

(51) Int Cl.:
***A61F 13/53*** (2006.01)   ***D04H 1/46*** (2012.01)

(21) Application number: **18864852.1**

(22) Date of filing: **02.10.2018**

(86) International application number:
**PCT/JP2018/036768**

(87) International publication number:
**WO 2019/069881 (11.04.2019 Gazette 2019/15)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.10.2017  JP 2017193877**
             **28.11.2017  JP 2017228430**

(71) Applicant: **Kao Corporation**
**Tokyo 103-8210 (JP)**

(72) Inventors:
• **TATSUMI, Yuta**
  **Haga-gun**
  **Tochigi 321-3497 (JP)**

• **MATSUI, Manabu**
  **Haga-gun**
  **Tochigi 321-3497 (JP)**
• **MOTEGI, Tomoyuki**
  **Haga-gun**
  **Tochigi 321-3497 (JP)**
• **KATO, Yuki**
  **Haga-gun**
  **Tochigi 321-3497 (JP)**

(74) Representative: **Vossius & Partner**
**Patentanwälte Rechtsanwälte mbB**
**Siebertstrasse 3**
**81675 München (DE)**

(54) **ABSORBENT AND ABSORBENT ARTICLE**

(57)    An absorbent member (10) according to the present invention includes: a plurality of fiber clusters (11) containing synthetic fibers (11F); and a plurality of absorbent fibers (12F). The fiber clusters (11) are entangled with each other, or the fiber clusters (11) and the absorbent fibers (12F) are entangled. Each of the fiber clusters (11) includes two opposing base surfaces (111) and a body surface (112) that connects the two base surfaces (111). The number of fiber end portions per unit area in the body surface (112) is greater than the number of fiber end portions per unit area in each of the base surfaces (111).

Fig. 1

**Description**

Technical Field

The present invention relates to an absorbent member for use in an absorbent article.

Background Art

An absorbent article such as a disposable diaper or a sanitary napkin is usually composed of a top sheet disposed so as to be positioned relatively closely to the skin of the wearer, a back sheet disposed so as to be positioned relatively far from the skin of the wearer, and an absorbent member disposed between the top sheet and the back sheet. Typically, the absorbent member is formed mainly of hydrophilic fibers (absorbent fibers) such as wood pulp, and often also includes absorbent polymer particles. The absorbent member for use in an absorbent article has major problems regarding improvements in various properties such as flexibility (cushioning properties), compression recovery, and shape retention.

A technique for improving an absorbent member is disclosed in, for example, Patent Literature 1. Patent Literature 1 discloses an absorbent member that includes thermoplastic resin fibers and cellulosic absorbent fibers, wherein the thermoplastic resin fibers are exposed on both the surface of the top sheet side of the absorbent member and the surface of the back sheet side of the absorbent member. Patent Literature 1 purports that the absorbent member disclosed therein is soft and is unlikely to be twisted because the thermoplastic resin fibers function as a skeleton for holding other components of the absorbent member such as the cellulosic absorbent fibers.

Also, Patent Literature 2 discloses an absorbent member including: fragments of nonwoven fabric including heat-fusible fibers, the heat-fusible fibers being bonded to each other to have a three-dimensional structure in advance of formation of the fragments; and hydrophilic fibers. The fragments of the nonwoven fabric having a three-dimensional structure are produced by cutting a non-woven fabric into small fragments using a cutting means such as a cutter mill. Due to this production method, the fragment has an indefinite shape as shown in Figs. 1 and 3 of Patent Literature 2, and does not substantially have a portion that can be regarded as a flat face. Patent Literature 2 discloses an absorbent member according to a preferred embodiment in which the fragments of the nonwoven fabric are thermally fused to each other. Patent Literature 2 purports that empty spaces are formed within the absorbent member since the fragments of the nonwoven fabric have a three-dimensional structure, and that restorability when moisture is absorbed is thus improved to thereby improve moisture absorption.

Also, Patent Literature 3 discloses a microweb comprising relatively dense microfiber nuclei with individual fibers or fiber bundles protruding therefrom, and also discloses that a non-woven web obtained by combining the microweb with wood pulp and absorbent polymer particles can be used as an absorbent member for use in an absorbent article. The microweb is produced by divellicating or tearing a sheet material web such as a non-woven fabric. Accordingly, as with the fragments of the nonwoven fabric disclosed in Patent Literature 2, it has an indefinite shape, and does not substantially have a portion that can be regarded as a flat face.

Citation List

Patent Literatures

Patent Literature 1: U.S. Patent Publication No. 2015/0366726
Patent Literature 2: U.S. Patent Publication No. 2010/0174259
Patent Literature 3: U.S. Patent No. 4813948

Summary of Invention

The present invention relates to an absorbent member including: a plurality of fiber clusters containing synthetic fibers; and a plurality of absorbent fibers. The fiber clusters are entangled with each other, or the fiber clusters and the absorbent fibers are entangled. Each of the fiber clusters includes two opposing base surfaces and a body surface that connects the two base surfaces. The number of fiber end portions per unit area in the body surface is greater than the number of fiber end portions per unit area in each of the base surfaces.

The present invention also relates to an absorbent member including: a plurality of fiber clusters containing synthetic fibers; and a plurality of absorbent fibers. The fiber clusters are entangled with each other, or the fiber clusters and the absorbent fibers are entangled. Each of the fiber clusters includes two opposing base surfaces and a body surface that connects the two base surfaces. Each of the fiber clusters includes an extended fiber bundle portion extending outward from the body surface and containing a plurality of fibers.

Brief Description of Drawings

[Fig. 1] Fig. 1 is a schematic perspective view of an absorbent member according to an embodiment of the present invention.

[Fig. 2] Fig. 2 is a diagram schematically showing a deformed state of the absorbent member shown in Fig. 1 when the absorbent member is compressed.

[Fig. 3] Figs. 3(a) and 3(b) are schematic perspective views of fiber clusters according to the present invention.

[Fig. 4] Fig. 4 is a diagram illustrating a method for producing a fiber cluster according to the present invention.

[Fig. 5] Fig. 5(a) is an electron microscopic image (captured at a magnification of 25 times) of an example of a fiber cluster according to the present invention, and Fig. 5(b) is a diagram schematically illustrating the fiber cluster imaged in the electron microscopic image as an example of a fiber cluster included in the absorbent member shown in Fig. 1.

[Fig. 6] Fig. 6 is a schematic perspective view of an apparatus for producing an absorbent member according to an embodiment of the present invention.

[Fig. 7] Fig. 7 is an enlarged side view of a second supply mechanism (production apparatus for fiber cluster) included in the production apparatus shown in Fig. 6.

Description of Embodiments

In the absorbent member disclosed in Patent Literature 1, a plurality of synthetic fibers included in the absorbent member are each independent, and thus do not form one single collective cluster. Accordingly, the effect of improving various properties is not sufficient. For this reason, when the absorbent member is used in an absorbent article, it may soon become twisted and may fit insufficiently. Such problems are significant particularly when a body fluid such as urine or menstrual blood is absorbed.

Regarding the synthetic fiber wads included in the absorbent members disclosed in Patent Literatures 2 and 3, they have an indefinite shape, and their shape and size are not uniform, as described above. For this reason, when the synthetic fiber wad is used together with wood pulp or the like, it is difficult to uniformly mix the synthetic fiber wads and the wood pulp, and a desired effect may not be obtained. Also, the synthetic fiber wads disclosed in the Patent Literatures are produced by cutting a non-woven fabric made mainly of synthetic fibers into small pieces, or by divellicating or tearing the non-woven fabric, and it is therefore assumed that the surface is randomly made rough. In an absorbent member that includes a plurality of synthetic fiber wads whose entire surface is rough, the plurality of synthetic fiber wads may be entangled with a relatively strong bonding force over the entire surface thereof. As a result, the degree of freedom of movement of each synthetic fiber wads is significantly limited, and the softness of the absorbent member may be impaired. Also, when all of the synthetic fiber wads included in the absorbent member are thermally fused as in a preferred embodiment of the absorbent member of Patent Literature 2, the movement of the synthetic fiber wads is limited. As a result, the hardness of the absorbent member as a whole increases, and various properties such as flexibility may be reduced.

Accordingly, the present invention relates to an absorbent member that has excellent cushioning properties and excellent compression recovery, is capable of flexibly deforming in response to an external force, and can provide improved wearing comfort and a better fit when it is used in an absorbent article, and an absorbent article that includes the absorbent member.

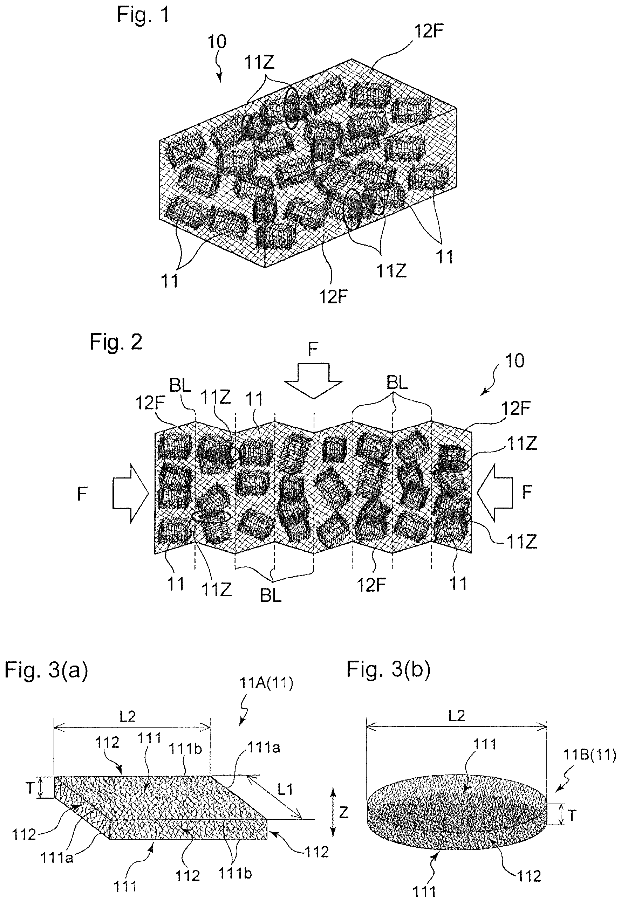

Hereinafter, the present invention will be described with reference to the drawings by way of a preferred embodiment. Fig. 1 shows an absorbent member 10 as an embodiment of an absorbent member of the present invention. The absorbent member 10 includes fiber clusters 11 composed of a plurality of fibers 11F, and absorbent fibers 12F.

The term "fiber cluster" herein refers to a fiber wad formed by collectively combining a plurality of fibers. A fiber cluster may be in the form of a small piece that has been cut out from, for example, a synthetic fiber sheet of a certain size. In particular, it is preferable to select a non-woven fabric as the synthetic fiber sheet, cut out a nonwoven fabric small piece with a predetermined size and shape from the non-woven fabric, and use the nonwoven fabric small piece as the fiber cluster.

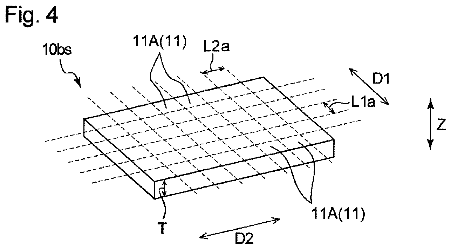

As described above, the fiber cluster in the form of a small piece from a sheet, which is a preferred embodiment of the fiber cluster in the present invention, is produced by cutting out a piece from a fiber sheet (preferably a non-woven fabric) that has a size larger than the piece, rather than by collecting a plurality of fibers and shaping them into a sheet-like small piece (see Fig. 4). The plurality of fiber clusters included in the absorbent member according to the present invention are each a fiber cluster in the form of a sheet-like small piece that have a high level of definite shape, unlike those produced by conventional techniques such as Patent Literatures 2 and 3.

In the absorbent member 10, a plurality of fiber clusters 11 are entangled with each other, or fiber clusters 11 and absorbent fibers 12F are entangled. In the absorbent member 10 according to the present embodiment, a plurality of fiber clusters 11 are entangled with and bonded to the constituent fibers (fibers 11F and 12F) of the absorbent member 10, thereby forming one large continuous fiber cluster. Also, a configuration is possible in which a plurality of fiber clusters 11 are entangled with each other while fiber clusters 11 and absorbent fibers 12F are also entangled and bonded. Furthermore, usually, a plurality of absorbent fibers 12F are also entangled with each other. At least some of the plurality of fiber clusters 11 included in the absorbent member 10 are entangled with other fiber clusters 11 or absorbent fibers 12F. In the absorbent member 10, all of the plurality of fiber clusters 11 included in the absorbent member 10 may be entangled with each other to form a single larger continuous fiber cluster, or a plurality of large continuous fiber clusters may exist together without bonding to each other.

The fiber clusters 11 have excellent flexibility and other properties. Accordingly, as a result of incorporating the fiber clusters 11 in an absorbent member, the resulting absorbent member inherently has excellent flexibility and other properties. In the absorbent member 10 according to the present invention, in addition to incorporating the fiber clusters 11, the fiber clusters 11 are entangled with and bonded to each other, or the fiber clusters 11 and the absorbent fibers 12F are also entangled with and bonded to each other. Accordingly, the absorbent member 10 responds even better to an external force, and thus has excellent flexibility, excellent cushioning properties, and excellent compression recovery. For example, when the absorbent member 10 according to the present invention is incorporated in an absorbent article, the absorbent article can flexibly deform in response to an external force (for example, the body pressure of the wearer wearing the absorbent article) received from various directions, and the absorbent article thus has a good fit to the body of the wearer.

Fig. 2 schematically shows a deformed state of the absorbent member 10 when the absorbent member 10 is compressed by an external force F. In the absorbent member 10 in which fiber clusters 11, which are fiber wads, and absorbent fibers 12F, which are not fiber wads, exist together, the absorbent member 10 is likely to bend particularly at boundaries BL (dotted lines shown in Fig. 2) between the fiber clusters 11 and the absorbent fibers 12F, due to the difference in rigidity between the fiber clusters 11 and the absorbent fibers 12F. The boundaries BL, which function as bending portions when the absorbent member 10 undergoes deformation, are usually present over the entire region of the absorbent member 10, and thus the absorbent member 10 can flexibly deform in response to any external force, and can also quickly return to its original state due to compression recovery of the fiber clusters 11 when the external force is removed. Such deformation/recovery properties of the absorbent member 10 are exhibited not only when the absorbent member 10 is compressed, but also when the absorbent member 10 is twisted. For example, when the absorbent member 10 is applied to an absorbent article such as a sanitary napkin, the absorbent member 10 is usually disposed between two thighs of the wearer wearing the absorbent article. Accordingly, the absorbent member 10 may be twisted about an imaginary rotation axis extending in the front-back direction of the wearer due to movement of the two thighs when the wearer walks. Even in such a case, because the absorbent member 10 has good deformation/recovery properties, the absorbent member 10 can easily recover from deformation in response to an external force that causes a twist from the two thighs. Accordingly, the absorbent article is unlikely to be twisted, and thus can provide a good fit to the body of the wearer.

As described above, in the absorbent member 10, fiber clusters 11 are entangled with each other, or fiber clusters 11 and absorbent fibers 12F are entangled with each other. As used herein, the term "entangled" and other related terms used to express that fiber clusters 11 or the like are entangled encompass the following configurations A and B.

Configuration A: fiber clusters 11 or the like are bonded to each other as a result of entanglement of constituent fibers 11F of the fiber clusters 11 with each other, rather than fusion thereof to each other.

Configuration B: fiber clusters 11 or the like are not bonded to each other when the absorbent member 10 is in a natural state (the state in which an external force is not applied), but when an external force is applied to the absorbent member 10, fiber clusters 11 or the like are bonded to each other as a result of entanglement of constituent fibers 11F with each other. As used herein, the expression "an external force is applied to the absorbent member 10" means, for example, the state in which a deformation force is applied to the absorbent member 10 in an absorbent article while worn.

As described above, in the absorbent member 10, some fiber clusters 11 are bonded to other fiber clusters 11 or absorbent fibers 12F by means of entanglement as in the configuration A, and some fiber clusters 11 are capable of being entangled with other fiber clusters 11 or absorbent fibers 12F as in the configuration B. Such bonding due to entanglement of fibers is an important point in further effectively exhibiting the above-described advantageous effects of the absorbent member 10. However, the absorbent member 10 preferably has the "entanglement" of the configuration A in view of shape retention. Bonding due to entanglement of fibers is formed only by entanglement of fibers, without an adhesive component or fusing, and thus, the degree of freedom of movement of individual entangling elements (fiber clusters 11 and absorbent fibers 12F) is high as compared with bonding due to "fusing fibers" as in Patent Literature 2, for example. Accordingly, the individual elements can move within a range where the wads formed of the elements can maintain its integrity. As described above, because a plurality of fiber clusters 11 included in the absorbent member 10 are relatively loosely bonded to each other, or because fiber clusters 11 and absorbent fibers 12F included in the absorbent member 10 are relatively loosely bonded, the absorbent member 10 has mild shape retention and can deform in response to exposure to an external force. Accordingly, high levels of shape retention, cushioning properties, and compression recovery are achieved.

However, as in the configuration B, not all bonding via the fiber clusters 11 in the absorbent member 10 need to be formed through "entanglement", and, other bonding conditions may be included in a part of the absorbent member 10, including joining via an adhesive.

In view of more reliably exhibiting the above-described advantageous effects of the absorbent member 10, the total number of "fiber clusters 11 that are bonded through entanglement", which correspond to the configuration A, and "fiber clusters 11 that are capable of being entangled", which correspond to the configuration B, preferably accounts for 50% or more, more preferably 70% or more, and even more preferably 80% or more, relative to the total number of fiber clusters 11 in the absorbent member 10.

From the same viewpoint, the number of fiber clusters 11 having "entanglement" of the configuration A preferably accounts for 70% or more, and particularly preferably 80% or more, of the total number of fiber clusters 11 having bonding portions to other fiber cluster 11 or absorbent fibers 12F.

A main feature of the absorbent member 10 is the outer shape of the fiber clusters 11. Fig. 3 shows two typical outer shapes of fiber clusters 11. A fiber cluster 11A shown in Fig. 3(a) has a quadrangular prism shape, more specifically, a rectangular parallelepiped shape. A fiber cluster 11B shown in Fig. 3(b) has a disc shape. The fiber clusters 11A and 11B are identical in having two opposing base surfaces (base planes) 111 and a body surface (lateral body surface) 112 that connects the two base surfaces 111. The base surfaces 111 and the body surface 112 are each a portion that has substantially no irregularities in terms of a level for an evaluation of the degree of unevenness on the surface of an article formed mainly of this type of fiber.

The rectangular parallelepiped fiber cluster 11A shown in Fig. 3(a) has six flat faces. Of the six faces, two opposing faces having the largest area correspond to the base surfaces 111, and the remaining four faces correspond to the body surfaces 112. The base surfaces 111 and the body surfaces 112 intersect each other, more specifically, they are orthogonal to each other.

The disc-shaped fiber cluster 11B shown in Fig. 3(b) has two opposing flat faces each in the form of a circul in a plan view and a curved peripheral surface that connects the two flat faces. The two flat faces correspond to the base surfaces 111, and the peripheral surface corresponds to the body surface 112.

The fiber clusters 11A and 11B are also identical in that the body surface 112 has a quadrangular shape, more specifically, a rectangular shape, in a plan view.

The plurality of fiber clusters 11 contained in the absorbent member 10 are different from the fragments of nonwoven fabric disclosed in Patent Literature 2 and the microweb disclosed in Patent Literature 3, which are fiber wads having an indefinite shape, in that each fiber cluster 11 is a "fiber wad having a definite shape" that has two opposing base surfaces 111 and a body surface 112 that connects the two base surfaces 111, as with the fiber clusters 11A and 11B shown in Fig. 3. In other words, when looking through a fiber cluster 11 arbitrarily selected from among the fiber clusters 11 contained in the absorbent member 10 (observed using, for example, an electron microscope), a plurality of perspective shapes are obtained with respect to one fiber cluster 11 because the perspective shape of the fiber cluster 11 varies depending on the viewing angle. Accordingly, each of the plurality of fiber clusters 11 contained in the absorbent member 10 has a specific perspective shape that has two opposing base surfaces 111 and a body surface 112 that connects the two base surfaces 111 as one of the plurality of perspective shapes. The plurality of the fragments of nonwoven fabric and the microweb respectively contained in the absorbent members disclosed in Patent Literatures 2 and 3 do not substantially have "faces" or "surfaces" like the base surfaces 111 and the body surface 112, or in other words, wide portions, and thus they have different outer shapes and thus do not have a "definite shape".

As described above, when the plurality of fiber clusters 11 contained in the absorbent member 10 are each a "fiber wad having a definite shape" defined by base surfaces 111 and one or more body surfaces 112, such fiber clusters 11 exhibit the improved uniform dispersibility in the absorbent member 10 as compared with the fiber wads having an indefinite shape as disclosed in Patent Literatures 2 and 3. Accordingly, the effects (the effects of improving the flexibility, cushioning properties, compression recovery and the like of the absorbent member) expected to be obtained by incorporating the fiber wads such as the fiber clusters 11 into the absorbent member 10 are exhibited stably. Also, particularly in the case of the fiber cluster 11 having a rectangular parallelepiped shape as shown in Fig. 3(a), the outer surface is composed of six faces, i.e., two base surfaces 111 and four body surfaces 112. Accordingly, the fiber cluster 11 having a rectangular parallelepiped shape as shown in Fig. 3(a) can have relatively more contact points with other fiber clusters 11 or absorbent fibers 12F than the disc-shaped fiber cluster 11 composed of three outer surfaces as shown in Fig. 3(b), and thus the former has enhanced entangling properties, which can lead to an improvement in shape retention and other properties.

In the fiber cluster 11, the total area of the two base surfaces 111 is preferably larger than the total area of the body surface(s) 112. That is, in the rectangular parallelepiped fiber cluster 11A as shown in Fig. 3(a), the sum of respective areas of the two base surfaces 111 is larger than the sum of respective areas of the four body surfaces 112, and in the disc-shaped fiber cluster 11B shown in Fig. 3(b), the sum of respective areas of the two base surfaces 111 is larger than the area of the body surface 112 that is the peripheral surface of the disc-shaped fiber cluster 11B. In each of the fiber clusters 11A and 11B, the base surfaces 111 are the surfaces that have the largest area among the plurality of surfaces thereof.

The fiber cluster 11, which is a "fiber wad having a definite shape" defined by two base surfaces 111 and one or more body surface(s) 112 that intersect the two base surfaces 111, can be provided by a production method different from conventional techniques. A preferred method for producing a fiber cluster 11 includes, as shown in Fig. 4, cutting a fiber sheet material 10bs (a sheet that has the same composition as that of the fiber cluster 11 and is larger than the fiber cluster 11) as a starting material into definite shapes using a cutting means such as a cutter. A plurality of fiber clusters 11 produced in this manner have a uniform definite shape and dimensions as compared with those produced by conventional techniques as disclosed in Patent Literatures 2 and 3. Fig. 4 is a diagram illustrating a method for producing rectangular parallelepiped fiber clusters 11A as shown in Fig. 3(a), and the dotted lines shown in Fig. 4 indicate cutting lines. The absorbent member 10 contains a plurality of fiber clusters 11 with a uniform shape and dimensions that are obtained by cutting a fiber sheet into a definite shape in such a manner. As described above, a non-woven fabric is preferably used as the fiber sheet material 10bs.

The rectangular parallelepiped fiber clusters 11A as shown in Fig. 3(a) are produced by cutting the fiber sheet material 10bs into predetermined lengths in a first direction D1 and a second direction D2 that intersects (more specifically, is orthogonal to) the first direction D1, as shown in Fig. 4. The directions D1 and D2 are each a predetermined direction in the surface direction of the sheet 10bs, and the sheet 10bs is cut along a thickness direction Z that is orthogonal to the surface direction. In a plurality of rectangular parallelepiped fiber clusters 11A obtained by cutting the fiber sheet material 10bs into so-called dice in such a manner, the cut surfaces (i.e., the surfaces that have come into contact with a cutting means such as a cutter when cutting) usually correspond to the body surfaces 112, and the non-cut surfaces (i.e., the surfaces that have not come into contact with the cutting means) correspond to the base surfaces 111. The base surfaces 111 are front and back surfaces of the sheet 10bs (the surfaces that are orthogonal to the thickness direction Z), and, as described above, the base surfaces 111 have the largest area among the plurality of surfaces of a fiber cluster 11A.

The description of the fiber cluster 11A given above can be basically applied to the disc-shaped fiber cluster 11B shown in Fig. 3(b). The only substantial difference thereof from the fiber cluster 11A is the cutting pattern on the fiber sheet material 10bs. In order to obtain fiber clusters 11B by cutting the sheet material 10bs into definite shapes, the sheet material 10bs may be cut into circular shapes according to the shape of the fiber clusters 11B in a plan view.

Also, the outer shape of the fiber cluster 11 is not limited to those shown in Fig. 3, and the base surfaces 111 and the body surface 112 may be non-curved flat surfaces as with the surfaces 111 and 112 shown in Fig. 3(a), or curved surfaces as with the body surface 112 (the peripheral surface of the disc shaped fiber cluster 11B) shown in Fig. 3(b). The base surfaces 111 and the body surface 112 may have the same shape and the same dimensions, and specifically, for example, the outer shape of the fiber cluster 11A may be a cubic shape.

As described above, the two types of surfaces (i.e., the base surface 111 and the body surface 112) of the fiber cluster 11 (11A or 11B) can be classified into a cut surface (body surface 112) created by cutting the fiber sheet material 10bs using a cutting means such as a cutter when producing the fiber cluster 11; and a non-cut surface (base surface 111), which is an original surface of the sheet 10bs and has not come into contact with the cutting means. Due to whether or not the surface is a cut surface, the body surface 112, which is a cut surface, has a feature such that the number of fiber end portions per unit area is greater than that in the base surface 111, which is a non-cut surface. As used herein, the term "fiber end portion" refers to the end of a constituent fiber 11F of the fiber cluster 11 in the lengthwise direction thereof. Normally, there are fiber end portions in the base surface 111, which is a non-cut surface; however, because the body surface 112 is a cut surface created by cutting the fiber sheet material 10bs, many fiber end portions that are the cut ends of the constituent fibers 11F formed through cutting are present in the entire body surface 112, or in other words, the number of fiber end portions per unit area is greater than that of the base surface 111.

The fiber end portions present on each surface of a fiber cluster 11 (the base surfaces 111 and the body surface 112) are useful for forming entanglements between the fiber cluster 11 and other fiber clusters 11 and absorbent fibers 12F included in the absorbent member 10. Also, in general, entangling properties can be further improved as the number of fiber end portions per unit area is increased, leading to an improvement in various properties of the absorbent member 10 such as shape retention. As described above, the number of fiber end portions on each surface of a fiber cluster 11 is not uniform, and the number of fiber end portions per unit area may satisfy the following magnitude relationship: "body surface 112 > base surface 111". Therefore, entanglement of a fiber cluster 11 with other fibers (other fiber clusters 11 and absorbent fibers 12F) varies depending on the surface of the fiber cluster 11, and the entangling properties of the body surface 112 are better than those of the base surface 111. Specifically, the bonding force due to entanglement with other fibers via the body surface 112 is stronger than the bonding force due to entanglement with other fibers via the base surface 111, and a difference can occur between the bonding force due to entanglement with other fibers via the base surface 111 and that via the body surface 112 within one fiber cluster 11.

As described above, a plurality of fiber clusters 11 included in the absorbent member 10 are entangled with other fibers (other fiber clusters 11 and absorbent fibers 12F) that are present in the periphery thereof through two types of bonding forces. With this configuration, the absorbent member 10 has both appropriate softness and appropriate strength (shape retention). When the absorbent member 10 having excellent properties as described above is used as an absorbent member for an absorbent article according to a conventional method, great wearing comfort can be given to the wearer of the absorbent article, and the damage to the absorbent member 10 disadvantageously given by an external force such as the body pressure of the wearer of the absorbent article can be effectively prevented.

In particular, in the fiber cluster 11 (11A or 11B) shown in Fig. 3, the total area of two base surfaces 111 is larger than the total area of the body surface 112, as described above. This means that the total area is larger in the base surfaces 111, which have a relatively smaller number of fiber end portions per unit area and therefore relatively poorer entangling properties with other fibers, than the body surface 112, which has contrary properties thereto. Accordingly, in the fiber cluster 11 (11A or 11B) shown in Fig. 3, entanglement with other peripheral fibers (other fiber clusters 11 and absorbent fibers 12F) is more likely to be limited, as compared with a fiber cluster in which fiber end portions are uniformly present over the entire surface. Also, even when the fiber cluster 11 is entangled with other peripheral fibers, the fiber cluster 11 is entangled with other peripheral fibers with a relatively weak bonding force. Therefore, it is unlikely to become a large mass, and thus excellent flexibility can be imparted to the absorbent member 10.

In contrast, the fragments of the nonwoven fabric disclosed in Patent Literature 2 and the microweb disclosed in Patent Literature 3 are produced by cutting a fiber sheet material into indefinite shapes using a cutting machine such as a mill cutter, as described above. For this reason, they are not fiber clusters in the form of sheet-like small pieces having a definite shape with "surfaces" like the base surfaces 111 and the body surface 112. In addition, at the time of production of fiber clusters, an external force is applied to all of the fiber clusters during cutting processing. Accordingly, fiber end portions of the constituent fibers are formed randomly over the entire fiber clusters, and thus the above-described advantageous effects provided by the fiber end portions are not sufficiently exhibited.

In view of ensuring the above-described advantageous effects provided by the fiber end portions, the ratio of $N_1$, which is the number of fiber end portions per unit area of the base surfaces 111 (non-cut surface), to $N_2$, which is the number of fiber end portions per unit area of the body surface 112 (cut surface), i.e., $N_1/N_2$, is preferably 0 or more and more preferably 0.05 or more, and preferably 0.90 or less and more preferably 0.60 or less, provided that $N_1 < N_2$. More specifically, $N_1/N_2$ is preferably 0 or more and 0.90 or less, and more preferably 0.05 or more and 0.60 or more.

The number $N_1$ of fiber end portions per unit area of the base surfaces 111 is preferably 0 fiber end portions/$mm^2$ or more, and more preferably 3 fiber end portions/$mm^2$ or more, and preferably 8 fiber end portions/$mm^2$ or less, and more preferably 6 fiber end portions/$mm^2$ or less.

The number $N_2$ of fiber end portions per unit area of the body surface 112 is preferably 5 fiber end portions/$mm^2$ or more, and more preferably 8 fiber end portions/$mm^2$ or more, and preferably 50 fiber end portions/$mm^2$ or less, and more preferably 40 fiber end portions/$mm^2$ or less.

The number of fiber end portions per unit area of the body surface 112 and that of the base surfaces 111 are determined according to the following method.

Method for determining the number of fiber end portions per unit area in each surface of fiber cluster

A sample of a fiber-containing member (fiber cluster) as an object is attached to a sample stage using a piece of double-sided paper tape (NICE TACK NW-15 available from Nichiban Co., Ltd.). Next, the sample is subjected to platinum coating. The coating is performed using an ion sputtering apparatus E-1030 (product name) available from Hitachinaka Seiki Co., Ltd., for a sputtering time of 120 seconds. A cut surface (a base surface or a body surface) of the sample is observed using an electron microscope JCM-6000 available from JEOL at a magnification of 100 times. In an image captured at a magnification of 100 times, a rectangular region with a length of 1.2 mm and a width of 0.6 mm is determined at an arbitrary location on the observed surface (a base surface or a body surface), and the viewing angle and other conditions are adjusted such that the area of the rectangular region accounts for 90% or more of the captured image. Then, the number of fiber end portions present in the rectangular region is counted. However, in some cases, in an image captured at a magnification of 100 times, the observed surface of the fiber cluster may be smaller than 1.2 mm × 0.6 mm, and thus the area of the rectangular region may account for less than 90% of the captured image. In such cases, the magnification is set to be more than 100 times, and then, the number of fiber end portions present in the rectangular region on the observed surface is counted in the manner described above. As used herein, "fiber end portions" that are to be counted refer to the lengthwise ends of the constituent fibers of a fiber cluster, and when portions other than the lengthwise ends (middle portions in the lengthwise direction) of the constituent fibers extend from the observed surface, the middle portions in the lengthwise direction are not counted. Then, the number of fiber end portions per unit area of the observed surface (a base surface or a body surface) of the fiber cluster is calculated using the following equation. For ten fiber clusters, the number of fiber end portions per unit area of the base surface and that of the body surface are counted in the same manner as described above, and the average value of the found values is defined as the number of fiber end portions per unit area of the target surface.

$$\text{Number of fiber end portions per unit area of a target surface (a base surface or a}$$
$$\text{body surface) of a fiber cluster (fiber end portions/mm}^2) = \text{number of fiber end portions in}$$
$$\text{a rectangular region (1.2} \times \text{0.6 mm) / area of the rectangular region (0.72 mm}^2)$$

In the case where the base surface 111 of the fiber cluster 11 has a rectangular shape in a plan view as with the fiber cluster 11A shown in Fig. 3(a), each short side 111a of the rectangular shape is preferably smaller than or equal to the thickness of the absorbent member 10 that contains the fiber cluster 11 (11A), in view of improving uniform dispersibility of the fiber cluster 11 in the absorbent member 10.
The ratio of the length of the short side 111a to the thickness of the absorbent member 10, former / latter, is preferably 0.03 or more, and more preferably 0.08 or more, and preferably 1 or less, and more preferably 0.5 or less.
The thickness of the absorbent member 10 is preferably 1 mm or more, more preferably 2 mm or more, and preferably 10 mm or less, and more preferably 6 mm or less. The thickness of the absorbent member 10 is measured using the following method.

Method for measuring thickness of absorbent member

A measurement object (absorbent member 10) is gently placed on a horizontal surface such that there are no wrinkles or buckling therein, and then the thickness of the measurement object is measured under a load of 5 cN/cm$^2$. Specifically, the thickness measurement is performed using, for example, a thickness gauge PEACOCK DIAL UPRIGHT GAUGES R5-C (available from OZAKI MFG. CO. LTD.). At this time, a plate (an acrylic plate with a thickness of about 5 mm) that has a circular or square shape in a plan view and is sized so as to apply a load of 5 cN/cm$^2$ on the measurement object is provided between the tip end of the thickness gauge and the cut measurement object, and then the thickness is measured. The thickness measurement is performed at ten points, and the average of the found values is calculated and defined as the thickness of the measurement object.
The dimensions and so on of each portion of the fiber cluster 11 (11A or 11B) are preferably set in the manner as described below. The dimensions of each portion of the fiber cluster 11 can be measured using an electron microscopic image or the like obtained during an operation of identifying the outer shape of the fiber cluster 11, which will be described later.
In the case where the base surface 111 has a rectangular shape in a plan view as shown in Fig. 3(a), the length of the short side 111a, L1, is preferably 0.1 mm or more, more preferably 0.3 mm or more, and even more preferably 0.5 mm or more, and preferably 10 mm or less, more preferably 6 mm or less, and even more preferably 5 mm or less.
The length of a long side 111b, L2, of the base surface 111 with a rectangular shape as viewed in a plan view is preferably 0.3 mm or more, more preferably 1 mm or more, and even more preferably 2 mm or more, and preferably 30 mm or less, more preferably 15 mm or less, and even more preferably 10 mm or less.
When the base surface 111 has the largest area among the plurality of surfaces of the fiber cluster 11 as shown in Fig. 3, the length of the long side 111b, L2, matches the maximum overall length of the fiber cluster 11, and the maximum overall length matches the diameter of the base surface 111, which has a circular shape in a plan view, of the disc shaped fiber cluster 11B.
The ratio of the length L1 of the short side 111a to the length L2 of the long side 111b, L1/L2, is preferably 0.003 or more, more preferably 0.025 or more, and preferably 1 or less, and more preferably 0.5 or less. In the present invention, the shape of the base surface 111 in a plan view is not limited to a rectangular shape as shown in Fig. 3(a), and may be a square shape, or in other words, the ratio of the lengths of two sides that are orthogonal to each other, L1/L2, may be 1.
The thickness T of the fiber cluster 11, or in other words, the length T between two opposing base surfaces 111 is preferably 0.1 mm or more, more preferably 0.3 mm or more, and preferably 10 mm or less, and more preferably 6 mm or less.
Also, in the absorbent member 10, it is preferable that the fiber clusters 11 are distributed densely and uniformly in the entire absorbent member 10 because the response to an external force is likely to be isotropic. From this viewpoint, in projected views in two directions of the absorbent member 10 that are orthogonal to each other, it is preferable that there are overlapping portions of a plurality of fiber clusters 11 in an arbitral unit region of 10 mm square. Reference numeral 11Z in Figs. 1 and 2 indicates overlapping portions of a plurality of fiber clusters 11. As used herein, the expression "projected views in two directions that are orthogonal to each other" typically include a projected view in the thickness direction of the absorbent member (or in other words, a projected view when the absorbent member is observed from the skin-facing surface or the non-skin-facing surface of the absorbent member) and a projected view in a direction orthogonal to the thickness direction (or in other words, a projected view when the absorbent member is observed from a side thereof).

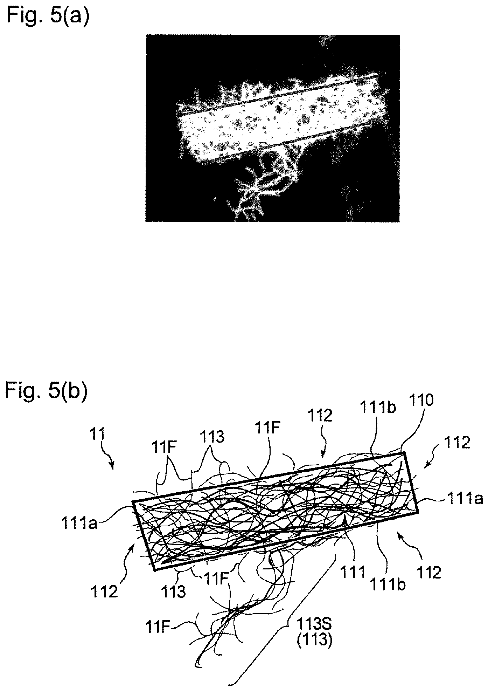

Fig. 5(a) is an electron microscopic image of a fiber cluster according to an embodiment of the present invention, and Fig. 5(b) is a diagram schematically illustrating the fiber cluster 11 imaged in the electron microscopic image. As shown in Fig. 5, the plurality of fiber clusters 11 included in the absorbent member 10 may each be composed of a main body portion 110 and an extended fiber portion 113 that includes a fiber 11F extending outward from the main body portion 110 and has a fiber density lower than that of the main body portion 110 (includes a small number of fibers per unit area). The absorbent member 10 may contain fiber clusters 11 that do not have extended fiber portions 113, or in other words, fiber clusters 11 that each consist of the main body portion 110. The extended fiber portion 113 may include either one type of the fiber end portion present on the base surface 111 or that on the body surface 112 of the fiber cluster 11 described above and the one type of the fiber end portion included in the extended fiber portion 113 extends outward from the corresponding surface of the fiber cluster 11.

As described above, the main body portion 110 is a portion defined by two opposing base surfaces 111 and a body surface 112 that connects the two base surfaces 111. The main body portion 110 is an essential portion of the fiber cluster 11 and forms the outer shape of the definite shape of the fiber cluster 11. Various properties of the fiber cluster 11 such as high flexibility, cushioning properties, and compression recovery essentially largely depend on the main body portion 110. On the other hand, the extended fiber portion 113 mainly contributes to improvement in entanglement between a plurality of fiber clusters 11 or entanglement between the fiber clusters 11 and absorbent fibers 12F in the absorbent member 10, to thereby provide a direct effect on improvement of the shape retention of the absorbent member 10, and also affects the uniform dispersibility of fiber clusters 11 in the absorbent member 10, to thereby indirectly reinforces the advantageous effects provided by the main body portion 110.

The main body portion 110 has a higher fiber density, or in other words, a higher number of fibers per unit area, than that of the extended fiber portion 113. Also, usually, the fiber density of the main body portion 110 itself is uniform. The mass proportion of the main body portion 110 relative to the total mass of the fiber cluster 11 is usually at least 40 mass% or more, preferably 50 mass% or more, more preferably 60 mass% or more, and even more preferably 85 mass% or more. The main body portion 110 and the extended fiber portion 113 can be distinguished from each other through the following operation of identifying the outer shape.

The operation of identifying the outer shape of the main body portion 110 of the fiber cluster 11 in the absorbent member 10 can be performed by checking the "boundaries" between the main body portion 110 and other portions while focusing on differences in fiber density (differences in the number of fibers per unit area), the type of fiber, the fiber diameter, and the like between the fiber cluster 11 and its peripheral portions. The main body portion 110 has a fiber density higher than that of the extended fiber portion 113 present in the surrounding region of the main body portion 110, and usually, the synthetic fibers that are the constituent fibers of the main body portion 110 are different from the absorbent fibers 12F (that are typically cellulosic fibers) in terms of characteristics and/or dimensions. Accordingly, even in an absorbent member 10 in which a plurality of fiber clusters 11 and absorbent fibers 12F are present in a mixed manner, boundaries as described above can be easily confirmed by focusing on the above differences. The boundaries identified in the above-described manner correspond to the peripheries (sides) of the base surface 111 and the body surface 112. Through such a boundary checking operation, the base surface 111 and the body surface 112 are identified, and eventually, the main body portion 110 is identified. The boundary checking operation can be performed by observing a target object (the absorbent member 10) from, where necessary, a plurality of viewing angles using an electron microscope. In particular, in the case where the fiber clusters 11 included in the absorbent member 10 are those in which "the total area of the two base surfaces 111 is larger than the total area of the body surface 112" as with the fiber clusters 11A and 11B shown in Fig. 3, particularly in the case where the base surfaces 111 have the largest area of the fiber cluster 11, such base surfaces 111 having the larger area can be identified relatively easily, and thus the operation of identifying the outer shape of the main body portion 110 can be performed smoothly.

As shown in Fig. 5, the extended fiber portion 113 is formed of one or more constituent fibers 11F from the main body portion 110 extending outward from at least one of the base surfaces 111 and the body surface 112, which define the outer surface of the main body portion 110. Fig. 5 shows a fiber cluster 11 as viewed in a plan view from the base surface 111 side (the surface having the largest area among the plurality surfaces of the fiber cluster 11), and the extended fiber portion 113 is formed of a plurality of fibers 11F extending from the body surface 112 that intersects the base surface 111.

There is no particular limitation on the form of the extended fiber portion 113. The extended fiber portion 113 may be formed of only one fiber 11F, or may be composed of a plurality of fibers 11F, such as an extended fiber bundle portion 113S described later. Also, the extended fiber portion 113 includes the lengthwise end portion of the fiber 11F extending from the main body portion 110. However, in addition to or instead of such a fiber end portion, the extended fiber portion 113 may include a portion other than the lengthwise ends (a middle portion in the lengthwise direction) of the fiber 11F. Specifically, in the fiber cluster 11, a portion other than the both lengthwise ends (i.e., the middle portion in the lengthwise direction) of the constituent fiber 11F may extend (protrude) in a loop outward from the main body portion 110 with the both lengthwise ends of the fiber 11F present in the main body portion 110. In this case, the extended fiber portion 113 includes the loop-shaped protruding portion of the fiber 11F. In other words, among extended fiber portions 113, the extended fiber portions 113 whose ends are exposed are one type of fiber end portion.

As described above, a main function of the extended fiber portion 113 is to cause a plurality of fiber clusters 11 included in the absorbent member 10 to entangle with each other, or to cause fiber clusters 11 and absorbent fibers 12F to entangle with each other. In general, when the extension length of the extended fiber portion 113 from the main body portion 110 increases, or when the thickness of the extended fiber portion 113 increases, or when the number of extended fiber portions 113 in one fiber cluster 11 increases, bonding between objects that are entangled via the extended fiber portion 113 increases, and thus the entangled objects are unlikely to come apart. Accordingly, the predetermined effects of the present invention are more stably exhibited.

In the case where the fiber cluster 11 is a fiber cluster obtained by cutting the fiber sheet material 10bs into definite shapes as shown in Fig. 4, a relatively large number of extended fiber portions 113 are present in the body surface 112 of the fiber cluster 11, which is a cut surface; on the other hand, in the base surface 111, which is a non-cut surface, there are no extended fiber portions 113, or if any, the number of extended fiber portions 113 is smaller than that in the body surface 112. Thus, the reason why the extended fiber portions 113 are concentrated in the body surface 112, which is a cut surface, is that most extended fiber portions 113 are "fluff" generated by cutting the fiber sheet material. Specifically, the entire body surface 112 formed by cutting the fiber sheet material IObs is rubbed by a cutting means such as a cutter when the fiber sheet material is cut, and thus fluff is likely to be generated from the constituent fiber 11F of the sheet IObs, or in other words, fluffing is likely to occur. On the other hand, the base surface 111, which is a non-cut surface, is not rubbed by the cutting means, and thus fluff (i.e., extended fiber portions 113) is unlikely to be formed.

The distance L1a (the distance in a first direction, see Fig. 4) and the distance L2a (the distance in a second direction, see Fig. 4) of cutting lines when the fiber sheet material IObs is cut is preferably 0.3 mm or more, and more preferably 0.5 mm or more, and preferably 30 mm or less, and more preferably 15 mm or less, in view of facilitating the formation of extended fiber portions 113 described above and ensuring dimensions necessary for the fiber cluster 11 to exhibit a desired effect.

As shown in Fig. 5, the fiber cluster 11 has an extended fiber bundle portion 113S containing a plurality of fibers 11F and extending outward from the main body portion 110, more specifically, from the body surface 112, as one type of extended fiber portion 113. At least one extended fiber portion 113 of the fiber cluster 11 may be the extended fiber bundle portion 113S. The extended fiber bundle portion 113S is an aggregation of a plurality of fibers 11F extending from the body surface 112, and is characterized in that the extension length from the body surface 112 is longer than that of an extended fiber portion 113. The extended fiber bundle portion 113S may be present on the base surface 111; however, typically as shown in Fig. 5, the extended fiber bundle portion 113S is present in the body surface 112. Thus, there is no extended fiber bundle portion 113S on the base surface 111, or if any, the number of extended fiber bundle portions 113S on the base surface 111 is smaller than that on the body surface 112. The reason for this is the same as the reason why the extended fiber portions 113 are present mainly in the body surface 112, which is a cut surface, as described above.

Because each fiber cluster 11 includes an extended fiber bundle portion 113S as described above that is a large-sized extended fiber portion 113 that is long and thick, entanglement between fiber clusters 11 or entanglement between fiber clusters 11 and absorbent fibers 12F is further increased. As a result, the effect of the present invention provided by the presence of fiber clusters 11 is obtained more stably. The extended fiber bundle portion 113S is easily formed by performing the above-described operation of cutting the fiber sheet material IObs under conditions where fluff is likely to be made (see Fig. 4).

The extension length of the extended fiber bundle portion 113S from the main body portion 110 (i.e., from the body surface 112 (cut surface)) is preferably 0.2 mm or more, and more preferably 0.5 mm or more, and preferably 7 mm or less, and more preferably 4 mm or less. The extension length of the extended fiber bundle portion 113S can be measured in the operation of identifying the outer shape of the fiber cluster 11 (boundary checking operation) described above. Specifically, for example, a fiber cluster 11 is placed on a transparent acrylic stage of a microscope available from Keyence Corporation (at a magnification of 50 times) and fixed thereto with a piece of double-sided adhesive tape available from 3M Company, and the outer shape of the fiber cluster 11 is identified through the outer shape identifying operation. After that, the length of extension of a fiber 11F extending from the outer shape is measured, and the measured length of extension is defined as the extension length of an extended fiber bundle portion 113S.

In the extended fiber bundle portion 113S, it is preferable that a plurality of constituent fibers 11F are thermally fused to each other. Such a thermally fused portion of the extended fiber bundle portion 113S usually has a larger overall thickness in a direction orthogonal to the lengthwise direction of the extended fiber bundle portion 113S (the overall thickness corresponds to the diameter in the case where the cross section of the thermally fused portion is circular) than that of other portions (non-thermally fused portions) of the extended fiber bundle portion 113S. Because the extended fiber bundle portion 113S has such a thermally fused portion, which may be also referred to as a "large-diameter portion", the strength of the extended fiber bundle portion 113S itself increases, and as a result, entanglement between fiber clusters 11 via the extended fiber bundle portion 113S or entanglement between fiber clusters 11 and absorbent fibers 12F via the extended fiber bundle portion 113S is further increased. Also, the extended fiber bundle portion 113S including a thermally fused portion is advantageous in that the extended fiber bundle portion 113S itself has improved strength, shape retention, and other properties not only when it is in a dry state, but also when it is in a wet state due to absorbing moisture. Thus, in the case where the absorbent member 10 is used in an absorbent article, the effects provided by the presence of fiber clusters 11 as described above can be exhibited stably due to the advantage described above, not only when the absorbent member 10 is in a dry state, but also when the absorbent member 10 is in a wet state due to absorbing a body fluid such as urine or menstrual blood of the wearer. The extended fiber bundle portion 113S that has a thermally fused portion can be produced by using a "fiber sheet that has a thermally fused portion of constituent fibers" as the fiber sheet material lObs in the step of producing fiber clusters 11 (in other words, the step of cutting the fiber sheet material lObs to obtain fiber clusters 11) as shown in Fig. 4.

The constituent fibers 11F of the fiber cluster 11 include synthetic fibers. The synthetic fibers used as the fibers 11F are preferably non-absorbent synthetic fibers. When the constituent fibers 11F of the fiber cluster 11 is non-absorbent fibers, the advantageous effects (the effect of improving shape retention, flexibility, cushioning properties, compression recovery, wear resistance, and the like) provided by the presence of fiber clusters 11 as described above can be exhibited stably not only when the absorbent member 10 is in a dry state but also when the absorbent member 10 is in a wet state due to absorbing moisture (a body fluid such as urine or menstrual blood). The amount of synthetic fibers used as the constituent fibers 11F of a fiber cluster 11 is preferably 90 mass% or more relative to the total mass of the fiber cluster 11, and most preferably 100 mass%, or in other words, it is most preferable that the fiber cluster 11 is made only of synthetic fibers. The advantageous effects provided by the presence of the fiber clusters 11 described above are more stably exhibited particularly when non-absorbent synthetic fibers are used as the constituent fibers 11F.

It will be readily understood by those skilled in the art that the term "absorbent" used herein means the ability to absorb moisture such as the ability of pulp to absorb moisture. Likewise, it will also be readily understood by those skilled in the art that thermoplastic fibers are non-absorbent. The level of moisture absorption of fibers such as synthetic fibers can be determined based on the value of moisture content measured according to the following method. The moisture content of the absorbent fibers is preferably 6% or more, and more preferably 10% or more. On the other hand, the moisture content of the non-absorbent fibers is preferably less than 6%, and more preferably less than 4%. When the moisture content is less than 6.0%, it is determined that the fibers are non-absorbent fibers. When the moisture content is 6.0% or more, it is determined that the fibers are absorbent fibers.

Method for measuring moisture content

Moisture content was calculated based on the moisture content testing method according to JIS P8203. Specifically, a fiber sample was left to stand in a testing room at a temperature of 40°C and a relative humidity of 80%RH for 24 hours. Then, the weight W (g) of the fiber sample before absolute drying was measured in the testing room. After that, the fiber sample was left to stand in an electric drier (for example, available from Isuzu Seisakusho, Co., Ltd.) at a temperature of 105 $\pm$ 2°C for one hour so as to subject the fiber sample to absolute drying. Then, in a testing room in a standard state of a temperature of 20 $\pm$ 2°C and a relative temperature of 65 $\pm$ 2%, the fiber sample wrapped in a plastic wrap (Saran Wrap, registered trademark) available from Asahi Kasei Corporation was placed in a glass desiccator (for example, available from Tech-Jam) containing Si silica gel (for example, available from Toyota Kako Co., Ltd.), and left to stand until the temperature of the fiber sample reached a temperature of 20 $\pm$ 2°C. After that, the constant weight W' (g) of the fiber sample was measured, and the moisture content of the fiber sample was determined using the following equation: Moisture content (%) = (W-W'/W') $\times$ 100.

In view of exhibiting excellent effects on shape retention, flexibility, cushioning properties, compression recovery, wear resistance, and other properties irrespective of whether the absorbent member 10 is in a dry state or in a wet state, the fiber cluster 11 preferably has a three-dimensional structure in which thermoplastic fibers are thermally fused to each other.

In order to obtain a fiber cluster 11 in which a plurality of thermally fused portions are three-dimensionally dispersed, the synthetic fibers used as the constituent fibers 11F of the fiber cluster 11 preferably include a plurality of thermoplastic fibers, and it is more preferable that the fiber cluster 11 consists of thermoplastic fibers. Also, when the constituent fibers 11F of the fiber cluster 11 include thermoplastic fibers, the above-described preferred form of the extended fiber bundle portion 113S, which has a thermally fused portion, can be obtained.

In order to obtain a fiber cluster 11 in which a plurality of thermally fused portions are three-dimensionally dispersed, the fiber sheet material lObs (see Fig. 4) may have the same configuration. The fiber sheet material lObs in which a plurality of thermally fused portions are three-dimensionally dispersed can be produced by, as described above, subjecting a web or non-woven fabric made mainly of thermoplastic fibers to heat treatment such as hot air treatment.

Examples of non-absorbent synthetic resin (thermoplastic resin) preferably used as the material of the constituent fiber 11F of the fiber cluster 11 include polyolefins such as polyethylene and polypropylene; polyesters such as polyethylene terephthalate; polyamides such as nylon 6 and nylon 66; polyacrylic acid, alkyl polymethacrylate, polyvinyl chloride, and polyvinylidene chloride. These may be used singly or in a combination of two or more. The fiber 11F may be a mono-fiber made of a single type of synthetic resin (thermoplastic resin) or a blend polymer in which two or more types of synthetic resins are mixed, or may be a composite fiber. As used herein, the composite fiber refers to a synthetic fiber (thermoplastic fiber) obtained by combining and spinning two or more types of synthetic resins of different components with a spinneret, the composite fiber having a structure in which the plurality of components are continuous in the lengthwise direction of the fiber and bonded to each other within the single fiber. The composite fiber may be in the form of sheath-core fiber or side-by-side fiber, and there is no particular limitation on the form of the composite fiber.

As the absorbent fiber 12F, any of absorbent fibers conventionally used as a material for forming an absorbent member for use in an absorbent article of this type can be used. Examples include: natural fibers including wood pulps such as softwood pulp and hardwood pulp and non-wood pulps such as cotton pulp and hemp pulp; and modified pulps such as cationized pulp and mercerized pulp. These may be used singly or in a combination of two or more. Out of the absorbent fibers, an absorbent cellulosic fiber is particularly preferably used.

In the absorbent member 10, there is no particular limitation on the mass ratio of the fiber clusters 11 and the absorbent fibers 12F included, and the mass ratio may be adjusted as appropriate according to a specific application of the absorbent member 10, the type of constituent fibers 11F of the fiber cluster 11, the type of absorbent fibers 12F, and other conditions. However, in view of reliably exhibiting the effects of the present invention, the mass ratio of the fiber clusters 11 and the absorbent fibers 12F included, the fiber clusters 11 / the absorbent fibers 12F, is preferably 20/80 to 80/20, and more preferably 40/60 to 60/40.

The amount of fiber cluster 11 included in the absorbent member 10 is preferably 20 mass% or more, and more preferably 40 mass% or more, and preferably 80 mass% or less, and more preferably 60 mass% or less relative to the total mass of the absorbent member 10 in a dry state.

The amount of absorbent fibers 12F included in the absorbent member 10 is preferably 20 mass% or more, and more preferably 40 mass% or more, and preferably 80 mass% or less, and more preferably 60 mass% or less relative to the total mass of the absorbent member 10 in a dry state.

The basis weight of the fiber cluster 11 in the absorbent member 10 is preferably 32 g/m$^2$ or more, more preferably 80 g/m$^2$ or more, and preferably 640 g/m$^2$ or less, and more preferably 480 g/m$^2$ or less.

The basis weight of the absorbent fibers 12F in the absorbent member 10 is preferably 32 g/m$^2$ or more, more preferably 80 g/m$^2$ or more, and preferably 640 g/m$^2$ or less, and more preferably 480 g/m$^2$ or less.

The absorbent member 10 may contain a component other than the fiber clusters 11 or the absorbent fibers 12F. The component other than the fiber cluster 11 or the absorbent fiber 12F may be, for example, an absorbent polymer. As the absorbent polymer, an absorbent polymer in the form of particles is generally used, or alternatively an absorbent polymer in the form of fibers may be used. In the case where a superabsorbent polymer in the form of particles is used, the shape may be any one of a spherical shape, a cluster shape, a round bale shape, and an indefinite shape. The average particle size of the absorbent polymer is preferably 10 $\mu$m or more, and more preferably 100 $\mu$m or more, and preferably 1000 $\mu$m or less, and more preferably 800 $\mu$m or less. As the absorbent polymer, a polymer or copolymer of acrylic acid or alkali metal salt of acrylic acid can be generally used. Examples thereof include polyacrylic acid and salts thereof; and polymethacrylic acid and salts thereof.

The amount of absorbent polymer contained in the absorbent member 10 is preferably 5 mass% or more, more preferably 10 mass% or more, and preferably 60 mass% or less, and more preferably 40 mass% or less relative to the total mass of the absorbent member 10 in a dry state.

The basis weight of the absorbent polymer in the absorbent member 10 is preferably 10 g/m$^2$ or more, and more preferably 30 g/m$^2$ or more, and preferably 100 g/m$^2$ or less, and more preferably 70 g/m$^2$ or less.

The basis weight of the absorbent member 10 may be adjusted as appropriate according to the application of the absorbent member 10. For example, when the absorbent member 10 is used as the absorbent member of an absorbent article such as a disposable diaper or a sanitary napkin, the basis weight of the absorbent member 10 is preferably 100 g/m$^2$ or more, and more preferably 200 g/m$^2$ or more, and preferably 800 g/m$^2$ or less, and more preferably 600 g/m$^2$ or less.

The absorbent member 10 that has a configuration as described above has excellent flexibility and excellent cushioning properties, also has excellent compression recovery, can deform quickly in response to an external force, and can quickly return to its original state when the external force is removed. The properties of the absorbent member as described above can be evaluated by using compression workload (WC) and compression recovery rate (RC) as a measure. The compression workload is a measure of cushioning properties of the absorbent member. The larger the WC value, the better the cushioning properties. The compression recovery rate is a measure that indicates the degree of recovery when the absorbent member is compressed and then released from the compression. The larger the RC value, the higher the compression recovery. Also, considering the liquid-absorbing and holding function of the absorbent member 10, the WC value and the RC value are preferably high not only when the absorbent member 10 is in a dry state but also when the absorbent member 10 is in a wet state due to absorbing a body fluid or the like. In order for the absorbent member 10 to have such properties when it is in a wet state, it is effective to use non-absorbent fibers such as thermoplastic fibers as the constituent fibers 11F of the fiber cluster 11, as described above.

Method for measuring compression workload (WC) and compression recovery rate (RC)

It is widely known that the compression workload (WC) and the compression recovery rate (RC) of the absorbent member 10 can be represented by found values obtained using KES (Kawabata Evaluation System) available from Kato Tech Co., Ltd. (reference document: Standardization and Analysis of Texture Evaluation (Second Edition), written by Sueo Kawabata, published on July 10, 1980). Specifically, the compression workload and the compression recovery rate can be measured using an automatic compression testing machine KES-FB3 AUTO-A available from Kato Tech Co., Ltd. The measurement procedure is as follows.

A sample having a quadrangular shape of 195 mm × 68 mm in a plan view (an absorbent member that is not wrapped in a core wrapping sheet; i.e., an absorbent core) is provided and attached to the testing table of the compression testing machine. Next, the sample is compressed between steel plates that have an area of 2 cm$^2$ and a circular flat face. The compression rate is 0.01 cm/sec, and the maximum compressive load is 490.2 mN/cm$^2$. In the recovery process as well, measurement is performed at the same rate. The compression workload (WC) is represented by the following equation. In the equation, $T_m$ represents the thickness under a load of 490.2 mN/cm$^2$ (4.9 kPa), $T_o$ represents the thickness under the load of 4.902 mN/cm$^2$ (49 Pa), and P represents the load during measurement (mN/ cm$^2$).

Also, the compression recovery rate (RC) is the ratio between the compression workload (WC) when the sample is compressed and the compression recovery workload (WC') when the sample recovers from the compression state to the original state, and is therefore represented by [WC'/WC] × 100.

[Math. 1]

$$WC = \int_{T_0}^{T_m} PdT$$

When the wearer of an absorbent article walks in which the absorbent member 10 configured as described above is incorporated, the absorbent member 10 twists and deforms in good response to an external force applied by two thighs of the wearer, and also quickly returns to its original state when the external force is removed. Such properties of the absorbent member can be evaluated in terms of torsion moment as a measure. As the value of torsion moment of the absorbent article measured using the following method is smaller, it can be determined that the absorbent member can more easily twist and deform, and is unlikely to creat creases even when it is incorporated in an absorbent article, and therefore that the absorbent article can provide an good fit. Also, for the same reason as for WC and RC described above, it is preferable that the absorbent member 10 can easily twist and deform irrespective of whether the absorbent member 10 is in a dry state or in a wet state.

An absorbent article including the absorbent member 10 in a dry state has a torsion moment of preferably 0.20 mN·m or more, and more preferably 0.30 mN·m or more, and preferably 1.30 mN·m or less, and more preferably 0.80 mN·m or less.

An absorbent article including the absorbent member 10 in a wet state has a torsion moment of preferably 0.20 mN·m or more, and more preferably 0.30 mN·m or more, and preferably 1.20 mN·m or less, and more preferably 0.80 mN·m or less.

Method for measuring torsion moment

As the measurement apparatus, a torsion tester as disclosed in Fig. 2 in JIS K 7244-2 is used. However, regarding measurement conditions, in particular the shape and dimensions of the sample, the shape and dimensions of the sample recommended by JIS K 7244-2 are not adopted herein because measurement herein is performed assuming that the absorbent member is to be used in an absorbent article. Instead, the shape and dimensions of the sample appropriate for the assumption of the use of the absorbent member in an absorbent article are used. Specifically, as the sample (the absorbent member in a dry state or in a wet state), a stack of a top sheet and the absorbent member is provided. The stack has a size of 120 mm × 70 mm and a rectangular shape in a plan view. As the stack, for example, a stack of an absorbent member and a member positioned on the skin-side of the wearer relative to the absorbent member when the absorbent article is worn may be used, and such a stack can be removed from a commercially available absorbent article. In the case where an additional member (for example, a member what is called a "sub-layer") is provided between the absorbent member and the top sheet, the stack containing the additional member is used as the sample. For the measurement, the thickness of the sample may be arbitrary but is preferably 1 to 10 mm. The sample is fixed at its both longitudinal ends to upper and lower clamps of the measurement apparatus. The sample is twisted by rotating both ends in opposite directions by 5 degrees about the longitudinal direction of the sample as the rotation axis, and the maximum value of torsion moment applied to the sample is taken as the found value. During measurement, a weight of 62 g is applied to a balance weight disclosed in Fig. 2 in JIS K 7244-2, and thus, measurement is performed under a tension of 62 gf in the longitudinal direction of the sample. The length between clamps is set to 100 mm under no tension.

Here, an "absorbent member in a dry state" that is a measurement sample in the above-described measurement method is provided by leaving the stack as the measurement sample to stand in an environment at a temperature of 23°C and a relative humidity of 50%RH for 24 hours. An "absorbent member in a wet state" that is a measurement sample in the above-described measurement method is provided by the following manner: the stack in a dry state is horizontally placed with its top sheet side (skin-facing surface side) facing upward; the top sheet is overlaid with a cylinder-equipped acrylic plate that has an opening for pouring with a diameter of 1 cm on the bottom; 5.0 g of defibrinated horse blood is poured through the opening; and the resulting state is maintained for one minute after pouring the defibrinated horse blood. The defibrinated horse blood to be poured into the stack as the measurement sample is defibrinated horse blood available from Nippon Bio-Test Laboratories Inc. and has an adjusted viscosity of 8 cp at a liquid temperature of 25°C. The viscosity is a viscosity measured using a viscometer TVB-1 0M available from Toki Sangyo Co., Ltd with a rotor L/Adp (rotor code 19) at a rotation speed of 30 rpm. Also, in measurement, the corresponding portions of the the measurement apparatus to the point on the stack at which defibrinated horse blood is poured and the peripheral portion thereof are covered with a piece of plastic wrap (Saran Wrap, registered trademark) available from Asahi Kasei Corporation that has been cut to be 4 cm × 4 cm in size, in order to prevent the measurement apparatus from getting wet.

The absorbent member according to the present invention is preferably used as a constituent member for constituting an absorbent article. As used herein, the absorbent article broadly encompasses articles that are used to absorb a body fluid (for example, urine, soft feces, menstrual blood, and sweat) discharged from a human body, including a so-called open-type disposable diaper with attachment tape, a pant-type disposable diaper, a sanitary napkin, a pair of sanitary pants, and an incontinence pad. An absorbent member used in an absorbent article typically includes a liquid absorbent core and a core wrapping sheet that is liquid permeable and covers the outer surface of the absorbent core. The absorbent member according to the present invention can be used as the absorbent core. As the core wrapping sheet, paper, non-woven fabric or the like can be used. The absorbent member 10 does not necessarily include a core wrapping sheet, and in this case, the absorbent core itself is used as the absorbent member 10 in an absorbent article.

An absorbent article that includes the absorbent member according to the present invention typically includes a liquid permeable top sheet that may come into contact with the wearer's skin when the absorbent article is worn, a liquid impermeable or moisture repellent back sheet, and a liquid retaining absorbent member provided between the top sheet and the back sheet. As the top sheet, any of various types of non-woven fabrics or porous synthetic resin sheets can be used. As the back sheet, a synthetic resin film made of polyethylene, polypropylene, polyvinyl chloride or the like, or a composite material of such a synthetic resin film and a non-woven fabric can be used. The absorbent article may further include various types of members according to the specific application of the absorbent article. Such members are known to those skilled in the art. For example, in the case where the absorbent article is to be applied to a disposable diaper or a sanitary napkin, one or more pairs of standing guards can be provided on the right and left side portions of the top sheet.

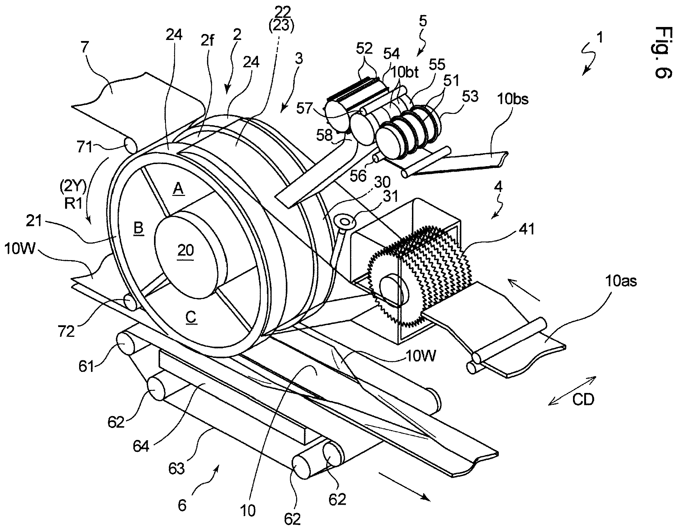

Hereinafter, a method for producing the absorbent member according to present invention will be described with reference to the drawings based on the method for producing the absorbent member 10 described above. Fig. 6 shows an overall configuration of a production apparatus (fiber stacking apparatus) 1 for producing an absorbent member 10. The production apparatus 1 includes a rotary drum 2 in which an accumulation recess portion 22 is formed in the outer peripheral surface 2f thereof and a duct 3 that internally has a flow path 30 for conveying a raw material of the absorbent member 10 to the outer peripheral surface 2f. The production apparatus 1 is configured such that the raw material is stacked in the accumulation recess portion 22 while the rotary drum 2 rotates about the rotation axis along a drum circumferential direction 2Y, the raw material being conveyed by an air flow (vacuum air) generated in the flow path 30 by suction from the inside of the rotary drum 2. A first supply mechanism 4 and a second supply mechanism 5 are connected to the duct 3 as supply mechanisms for supplying the raw material (fiber material) of the absorbent member 10. Under the rotary drum 2, a vacuum conveyor 6 is provided, and the vacuum conveyor 6 is for receiving a stacked fiber material (stacked raw material) released from the accumulation recess portion 22, i.e., an absorbent member 10, and conveying the received absorbent member 10 to the next step. On the side opposite to the duct 3 across the rotary drum 2, a pressing belt 7 for pressing the stacked fiber material in the accumulation recess portion 22 is provided, the pressing belt 7 extending along the outer peripheral surface 2f of the rotary drum 2. The pressing belt 7 is an endless and air permeable or non-air permeable belt that is looped around a roller 71 and a roller 72 and is pulled along with the rotation of the rotary drum 2.

The rotary drum 2 includes a cylindrical drum main body 20 that is a metal rigid body and an outer peripheral member 21 that is provided so as to overlap the outer peripheral portion of the drum main body 20 and provides the outer peripheral surface 2f of the rotary drum 2. The outer peripheral member 21 receives motive power from a power generator such as a motor, and rotates about the horizontal rotation axis in a direction R1 along the drum circumferential direction. However, the drum main body 20 provided on the inner side of the outer peripheral member 21 is fixed and thus does not rotate. Two opposite ends of the drum main body 20 in the drum width direction are each hermetically sealed with a side wall and a sealing member such as felt, which are not shown in the diagrams.

The outer peripheral member 21 includes an air permeable porous plate 23 that forms the bottom portion of the accumulation recess portion 22 (i.e., the fiber stacking surface), and a less air permeable or non-air permeable pattern forming plate 24 that forms a portion other than the fiber stacking surface in the outer peripheral surface 2f of the rotary drum 2. In the production apparatus 1, the pattern forming plate 24 has an annular shape extending continuously over the total length of the drum circumferential direction 2Y. A pair of pattern forming plates 24 and 24 are provided at opposite end portions in the rotation axis direction of the rotary drum 2, and the porous plate 23 is provided between the pair of pattern forming plates 24 and 24.

The porous plate 23 is an air permeable plate that transmits an air flow generated by suction from the inside of the apparatus (the inside of the rotary drum 2) to the outside of the apparatus (the outside of the rotary drum 2), retains the raw material transmitted by the air flow thereon without allowing the raw material to pass therethrough, and allows only air to pass therethrough. A plurality of air suction holes extending through the porous plate 23 in the thickness direction are formed over the entire porous plate 23, and the air suction holes function as the holes allowing the air flow to pass therethrough while the accumulation recess portion 22 passes over the space where the negative pressure is maintained within the rotary drum 2. As the porous plate 23, for example, a mesh plate made of a metal or a resin, or a metal or resin plate in which a plurality of pores are formed through etching or punching, or the like can be used. As the pattern forming plate 24, for example, a metal plate made of stainless steel or aluminum, a resin plate, or the like can be used.

As shown in Fig. 6, the interior of the drum main body 20 is divided into a plurality of spaces A, B, and C in the drum circumferential direction 2Y. A pressure reducing mechanism (not shown) that reduces the internal pressure is connected to the drum main body 20. The pressure reducing mechanism includes an air discharge pipe (not shown) connected to a side wall (not shown) of the drum main body 20 and an air discharge fan (not shown) connected to the air discharge pipe. The plurality of spaces A, B, and C in the drum main body 20 are independent of each other, and the negative pressures (suction forces) of the plurality of spaces can be independently controlled by the pressure reducing mechanism.

In the rotary drum 2, a predetermined area in the drum circumferential direction 2Y (specifically, the space A whose outer peripheral portion is covered by the duct 3) is a fiber stacking zone, in which the fiber as the raw material can stack by suction from the inner side thereof. When the outer peripheral member 21 is rotated about the rotation axis with the space A kept at a negative pressure, the negative pressure in the space A acts on the bottom portion (porous plate 23) of the accumulation recess portion 22 to thereby suck air through the plurality of air suction holes formed in the bottom portion while the accumulation recess portion 22 formed in the outer peripheral member 21 passes over the space A. As a result of air suction through the air suction holes, the raw material conveyed through a supply path 32 of the duct 3 is guided to the accumulation recess portion 22, and stacked on the bottom portion of the accumulation recess portion 22. On the other hand, the pressure in the space B of the rotary drum 2 is usually set to a negative pressure lower than that in the space A or zero pressure (atmospheric pressure), and the pressure in the space C is set to zero pressure or a positive pressure because the space C is an area that includes a zone for transfeing the stacked fiber material in the accumulation recess portion 22 and front and back zones thereof.

The vacuum conveyor 6 includes an endless air permeable belt 63 looped around a driving roller 61 and a driven roller 62, and a vacuum box 64 that is provided at a position opposing to the area where the space C of the rotary drum 2 is present with the air permeable belt 63 interposed therebetween. A core wrapping sheet 10W is introduced onto the air permeable belt 63, and the stacked fiber material as the absorbent member 10 released from the accumulation recess portion 22 is transferred on the core wrapping sheet 10W.

As shown in Fig. 6, the duct 3 extends continuously over the rotary drum 2 from the first supply mechanism 4, and has an upstream opening and a downstream opening (rotary drum 2 side opening) in the supply direction of the raw material. The flow path 30 for conveying the raw material extends between the upstream opening and the downstream opening. A polymer spray pipe 31 for supplying absorbent polymer particles to the flow path 30 is provided in the top plate of the duct 3. In the case where absorbent polymer particles are to be contained in the absorbent member 10, the polymer spray pipe 31 is used.

As described above, the absorbent member 10 contains two types of fiber materials, i.e., fiber clusters 11 and absorbent fibers 12F. Accordingly, the production apparatus 1 includes a first supply mechanism 4 (production apparatus for absorbent fibers) that supplies absorbent fibers 12F into the duct 3 and a second supply mechanism 5 (production apparatus for fiber clusters) that supplies fiber clusters 11 into the duct 3, as a fiber material supply mechanism.

The first supply mechanism 4 is provided at the opening opposite to the rotary drum 2 side of the duct 3. The first supply mechanism 4 has the same configuration as the fiber material supply mechanism of the fiber stacking apparatus for this type of pulp fiber or the like, and includes a defibring machine 40 for defibring a strip-shaped fiber sheet material 10as in which a plurality of absorbent fibers 12F are accumulated.

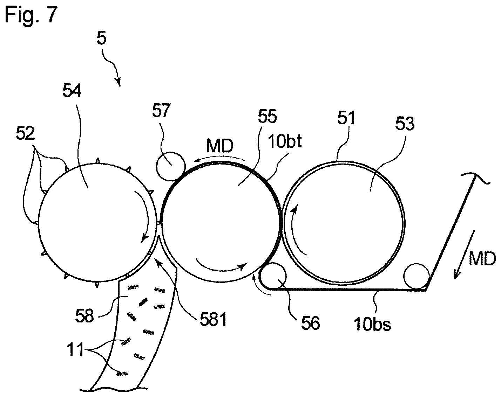

Fig. 7 is a schematic enlarged view of the second supply mechanism 5. The second supply mechanism 5 is an apparatus for producing and supplying rectangular parallelepiped-shaped fiber clusters 11 such as the fiber cluster 11A shown in Fig. 3(a), and the apparatus performs the step of cutting a strip-shaped fiber sheet material lObs made of the constituent fibers 11F for the fiber cluster 11 in a predetermined length in two directions (first direction D1 and second direction D2) that intersect each other as shown in Fig. 4. The second supply mechanism 5 includes a first cutter roller 53 for cutting an item to be cut (the fiber sheet material lObs) in the first direction D1, a second cutter roller 54 for cutting the item to be cut in the second direction D2, and a receiving roller 55 provided between the two rollers 53 and 54. The three rollers 53, 54, and 55 are configured to rotate in opposite directions to each other, with their outer peripheral surfaces facing each other, while their rotation axes are provided in parallel. Cutter blades 51 and 52 are provided on the outer peripheral surfaces of the cutter rollers 53 and 54, respectively, whereas no cutter blade is provided on the outer peripheral surface of the receiving roller 55. Thus the outer peripheral surface of the receiving roller 55 is smooth. In the vicinity of the outer peripheral surface of the receiving roller 55, a guide roller 56, the first cutter roller 53, a guide roller 57, and the second cutter roller 54 are provided in this order from the upstream side in the rotation direction.

"First direction D1", which is one of the cutting directions of the fiber sheet material lObs, corresponds to a conveyance direction MD of the fiber sheet material lObs in the second supply mechanism 5, and the angle formed between the first direction D1 and the conveyance direction MD is less than 45 degrees. In the embodiment shown in the diagram, the first direction D1 and the conveyance direction MD match each other, and the angle formed between the first direction D1 and the conveyance direction MD is zero.

Likewise, "second direction D2", which is the other one of the cutting directions of the fiber sheet material lObs, corresponds to a direction that intersects the first direction D1. In the embodiment shown in the diagram, the first direction D1 (the conveyance direction MD) and the second direction D2 are orthogonal to each other, and the angle formed by the directions D1 and D2 is 90 degrees.

Also, the direction indicated by reference sign CD in Fig. 6 is a direction that is orthogonal to the conveyance direction MD, and parallel to the rotation axes of the rotary drum 2 and various rollers of the production apparatus 1. In the embodiment shown in the diagram, the direction CD matchs the width directions (the direction orthogonal to the longitudinal direction) of the absorbent member 10 that is a stacked fiber material in an elongated strip-shape as well as the fiber sheet materials 10as and lObs each in an elongated strip-shape.

As shown in Figs. 6 and 7, on the outer peripheral surface of the first cutter roller 53, a plurality of cutter blades 51 extending in the circumferential direction of the roller 53 (first direction D1) are provided at a predetermined interval in the rotation axis direction of the roller 53, i.e., a CD direction (second direction D2).

On the outer peripheral surface of the second cutter roller 54, a plurality of cutter blades 52 extending in the rotation axis direction of the roller 54, i.e., a CD direction (second direction D2), are provided at a predetermined interval in the circumferential direction of the roller 54, i.e., the conveyance direction MD (first direction D1).

The method for producing an absorbent member 10 using the production apparatus 1 configured as described above is basically the same as a known method for producing an absorbent member using a fiber stacking apparatus having a similar configuration. Specifically, as shown in Fig. 6, a stacked fiber material is obtained in the following manner: absorbent fibers 12F obtained by defibring the fiber sheet material 10 as in the first supply mechanism 4 are conveyed to the rotary drum 2 using an air flow (vacuum air) generated in the flow path 30 of the duct 3 by suction from the inside of the rotary drum 2 while the rotary drum 2 is rotated in the direction R1 about the rotation axis along the drum circumferential direction 2Y, and the fibers are thus stacked in the accumulation recess portion 22 in the region where the space A is provided. The stacked fiber material is the absorbent member 10. The absorbent member 10 in the accumulation recess portion 22 passes through the region where the space A is provided (a portion of the outer peripheral surface 2f of the rotary drum 2 covered with the duct 3) along with the rotation of the outer peripheral member 21, and the absorbent member 10 is pressed by the pressing belt 7 when the absorbent member 10 is introduced to the region where the space B is provided, and conveyed to the vicinity of the vacuum conveyor 6. After that, the absorbent member 10 is released from the accumulation recess portion 22 to transfer onto a core wrapping sheet 10W introduced to the vacuum conveyor 6, and the the absorbent member 10 is then covered with the core wrapping sheet 10W.

The method for producing an absorbent member 10 is characteristic in that the method further includes, in addition to the basic steps described above, a step of producing and supplying fiber clusters 11 in the second supply mechanism 5. Specifically, as shown in Figs. 6 and 7, a main feature of the method for producing the absorbent member 10 is including: a step of cutting a strip-shaped fiber sheet material lObs in which a plurality of fibers 11F are accumulated in the first direction D1 (conveyance direction MD) extending along the longitudinal direction of the fiber sheet material lObs so as to obtain a plurality of strip-shaped narrow sheets 10bt and then cutting each of the plurality of narrow sheets 10bt in the second direction D2 (CD direction) that intersects the first direction D1 (is orthogonal to the first direction D1 in the embodiment shown in the diagram) so as to form fiber clusters 11; and a step of mixing the thus obtained fiber clusters 11 with absorbent fibers 12F separately provided.

In the second supply mechanism 5, a strip-shaped fiber sheet material lObs is first cut in a first direction D1, which is the longitudinal direction of the sheet lObs and is also the conveyance direction MD, between the first cutter roller 53 and the receiving roller 55, so as to produce a plurality of narrow sheets lObt extending in the direction D1, and the plurality of strip-shaped narrow sheets 10bt are then cut in a second direction D2, which is the width direction (CD direction) orthogonal to the longitudinal direction of the narrow sheets 10bt, between the receiving roller 55 and the second cutter roller 54 (cutting step). By cutting the strip-shaped fiber sheet material lObs sequentially in the first direction D1 and the second direction D2 orthogonal to the first direction D1 in this way, the fiber sheet material lObs is cut into so-called dice as shown in Fig. 5, and a plurality of fiber clusters 11 are thus obtained. The outer shape of the main body portion 110 of each of the plurality of fiber clusters 11 produced in the above-described manner is a rectangular parallelepiped shape as shown in Fig. 3(a).

The plurality of fiber clusters 11 produced by cutting the fiber sheet material lObs are supplied to the flow path 30 of the duct 3 via a suction nozzle 58, and mixed with absorbent fibers 12F scattered in the flow path 30 from the first supply mechanism 4 toward the rotary drum 2, and stacked in the accumulation recess portion 22 together with the absorbent fibers 12F (mixing step). The suction nozzle 58 has an opening at each of two opposite lengthwise ends thereof. The opening 581 that is one of the openings is located near the point at which the second cutter roller 54 and the receiving roller 55 are closest to each other, and the suction nozzle 58 is in communication with the flow path 30 of the duct 3 via the other opening (not shown). The plurality of fiber clusters 11 produced between the second cutter roller 54 and the receiving roller 55 are drawn into the suction nozzle 58 via the opening 581, and supplied into the duct 3. In the production apparatus 1, the connection of the suction nozzle 58 to the duct 3 is located between the rotary drum 2 and the first supply mechanism 4 and closer to the rotary drum 2 relative to the polymer spray pipe 31, as shown in Fig. 6.

Hereinabove, the present invention has been described by way of an embodiment of the present invention. However, the present invention is not limited thereto, and can be modified as appropriate.

For example, in the absorbent member according to the present invention, the fiber clusters are not necessarily dispersed uniformly in the entire absorbent member, and may be unevenly distributed. An absorbent member of an examplary embodiment in which fiber clusters are unevenly distributed may be an absorbent member that has a stack structure composed of a layer including mainly fiber clusters and a layer including mainly absorbent fibers.

Also, all of the fiber clusters (synthetic fiber wads) in the absorbent member according to the present invention are not necessarily fiber wads having a definite shape like fiber clusters 11. A very small number of fiber wads having an indefinite shape may be contained in addition to the fiber wads having a definite shape without departing from the gist of the present invention. With respect to the embodiment of the present invention, the following additional statements are also disclosed.

1. An absorbent member comprising:

   a plurality of fiber clusters containing synthetic fibers; and
   a plurality of absorbent fibers,
   wherein the fiber clusters are entangled with each other, or the fiber clusters and the absorbent fibers are entangled,
   each of the fiber clusters includes two opposing base surfaces and a body surface that connects the two base surfaces, and
   the number of fiber end portions per unit area in the body surface is greater than the number of fiber end portions per unit area in each of the base surfaces.

2. The absorbent member as set forth in claim 1,
wherein a total area of the two base surfaces is larger than a total area of the body surface.
3. The absorbent member as set forth in claim 1 or 2,
wherein each of the fiber clusters includes an extended fiber bundle portion extending outward from the body surface and containing a plurality of fibers.
4. The absorbent member as set forth in clause 3,
wherein the extended fiber bundle portion includes a thermally fused portion in which a plurality of fibers are thermally fused to each other.

5. The absorbent member as set forth in clause 3 or 4,

wherein each of the fiber clusters includes a main body portion defined by the base surfaces and the body surface, and an extension length of the extended fiber bundle portion from the main body portion, preferably an extension length of the extended fiber bundle portion from the body surface is 0.2 mm or more and 7 mm or less, preferably 0.5 mm or more and 4 mm or less.

6. The absorbent member as set forth in any one of clauses 1 to 5,

wherein an outer shape of each of the fiber clusters is a rectangular parallelepiped shape or a disc shape.

7. The absorbent member as set forth in any one of clauses 1 to 6,

wherein each of the base surfaces has a rectangular shape in a plan view, and a short side of the rectangular shape is smaller than a thickness of the absorbent member.

8. The absorbent member as set forth in clause 7,

wherein a ratio of a length of the short side of each of the base surfaces to the thickness of the absorbent member, former / latter, is 0.08 or more and 0.5 or less.

9. The absorbent member as set forth in clause 7 or 8,

wherein the length of the short side of each of the base surfaces is 0.3 mm or more and 10 mm or less, preferably 0.5 mm or more and 6 mm or less.

10. The absorbent member as set forth in any one of clauses 7 to 9,

wherein the length of a long side of each of the base surfaces is 0.3 mm or more and 30 mm or less, preferably 2 mm or more and 15 mm or less.

11. The absorbent member as set forth in any one of clauses 1 to 10,

wherein, in projected views in two directions of the absorbent member that are orthogonal to each other, there are overlapping portions of the plurality of fiber clusters in an arbitral unit region of 10 mm square.

12. The absorbent member as set forth in any one of clauses 1 to 11,

wherein each of the fiber clusters contains a plurality of thermoplastic fibers as the synthetic fibers, and has a three-dimensional structure in which the plurality of thermoplastic fibers are thermally fused to each other.

13. The absorbent member as set forth in any one of clauses 1 to 12,

wherein a mass ratio of the fiber clusters and the absorbent fibers included, former / latter, is 20/80 to 80/20.

14. The absorbent member as set forth in any one of clauses 1 to 13,

wherein, in the absorbent member, some fiber clusters are bonded to other fiber clusters or the absorbent fibers by means of entanglement, and some fiber clusters are capable of being entangled with other fiber clusters or the absorbent fibers.

15. The absorbent member as set forth in clause 14,

wherein the total number of the fiber clusters bonded through entanglement and the fiber clusters capable of being entangled accounts for preferably 50% or more, more preferably 70% or more, even more preferably 80% or more relative to the total number of the fiber clusters in the absorbent member.

16. The absorbent member as set forth in any one of clauses 1 to 15,

wherein the number of the fiber clusters having bonding portions to other fiber clusters or the absorbent fibers through entanglement of fibers accounts for preferably 70% or more, more preferably 80% or more of the total number of the fiber clusters having bonding portions to other fiber clusters or the absorbent fibers.

17. The absorbent member as set forth in any one of clauses 1 to 16,

wherein the fiber clusters are derived from a nonwoven fabric.

18. The absorbent member as set forth in any one of clauses 1 to 17,

wherein a ratio of the number $N_1$, of the fiber end portions per unit area in the base surfaces to the number $N_2$ of the fiber end portions per unit area in the body surface, $N_1/N_2$, is 0 or more and 0.90 or less, preferably 0.05 or more and 0.60 or less.

19. The absorbent member as set forth in any one of clauses 1 to 18,

wherein the number of fiber end portions per unit area in the base surfaces is 0 fiber end portions/mm$^2$ or more and 8 fiber end portions/mm$^2$ or less, preferably 3 fiber end portions/mm$^2$ or more and 6 fiber end portions/mm$^2$ or less.

20. The absorbent member as set forth in any one of clauses 1 to 19,

wherein the number of fiber end portions per unit area in the body surface is 5 fiber end portions/mm$^2$ or more and 50 fiber end portions/mm$^2$ or less, preferably 8 fiber end portions/mm$^2$ or more and more preferably 40 fiber end portions/mm$^2$ or less.

21. An absorbent member comprising:

   a plurality of fiber clusters containing synthetic fibers; and
   a plurality of absorbent fibers,
   wherein the fiber clusters are entangled with each other, or the fiber clusters and the absorbent fibers are entangled,

each of the fiber clusters includes two opposing base surfaces and a body surface that connects the two base surfaces, and

each of the fiber clusters includes an extended fiber bundle portion extending outward from the body surface and containing a plurality of fibers.

22. The absorbent member as set forth in clause 21,
wherein a total area of the two base surfaces is larger than a total area of the body surface.
23. An absorbent article comprising the absorbent member as set forth in any one of clauses 1 to 22.
24. The absorbent article as set forth in clause 23,
wherein the absorbent article in a dry state has a torsion moment of 0.20 mN·m or more and 1.30 mN·m or less, preferably 0.30 mN·m or more and 0.80 mN·m or less.
25. The absorbent article as set forth in clause 23 or 24,
wherein the absorbent article in a wet state has a torsion moment of 0.20 mN·m or more and 1.20 mN·m or less, preferably 0.30 mN·m or more and 0.80 mN·m or less.

Examples

Hereinafter, the present invention will be described in more detail by way of examples, but the present invention is not limited thereto.

Examples 1 to 4

Absorbent members were produced according to the production method described above using an apparatus for producing absorbent members that had the same configuration as the production apparatus 1 shown in Fig. 6. As the fiber sheet material for fiber clusters, an air-through non-woven fabric was used that contained, as a constituent fiber, hydrophobic thermoplastic fibers (a non-absorbent fiber with a fiber diameter of 18 $\mu$m) made of polyethylene resin and polyethylene terephthalate resin and had a basis weight of 21 g/m$^2$ (a fiber sheet that had thermally fused portions of constituent fibers). As the absorbent fiber, a needle bleached kraft pulp (NBKP) with a fiber diameter of 22 $\mu$m was used. The fiber clusters (synthetic fiber wads with a definite shape) used in the absorbent member each had a rectangular parallelepiped main body portion as shown in Fig. 3(a). The base surface 111 having a rectangular shape in a plan view had a short side 111a of 0.8 mm and a long side 111b of 3.9 mm, and the fiber clusters had a thickness T of 0.6 mm. Also, the number of fiber end portions per unit area in the base surface 111 was 3.2 fiber end portions/mm$^2$, and the number of fiber end portions per unit area of the body surface 112 was 19.2 fiber end portions/mm$^2$.

Comparative Example 1

The absorbent member of a commercially available sanitary napkin (product name: Tanom Pew Slim 23 cm, available from Unicharm Corporation) was used as the absorbent member of Comparative Example 1. The absorbent member of Comparative Example 1 was made of a mixture of synthetic fibers and cellulosic fibers (absorbent fibers), and did not contain any fiber clusters.

Comparative Example 2

An absorbent member was produced in the same manner as in Examples 1 to 4, except that small pieces of nonwoven fabric with an indefinite shape were used as fiber clusters, and that a hot air treatment was performed on the absorbent member so as to thermally fuse the small pieces of nonwoven fabric in the absorbent member to each other. In the hot air treatment on the absorbent member, the mixed aggregation (length 210 mm $\times$ width 66 mm) of the small pieces of nonwoven fabric and the pulp fibers was left to stand in an electric drier (for example, available from Isuzu Seisakusho, Co., Ltd.) at a temperature of 140°C for 30 minutes so as to thermally fuse the small pieces of nonwoven fabric to each other. The small pieces of nonwoven fabric with an indefinite shape used here were produced by tearing an air-through non-woven fabric that is the same as those used in Examples 1 to 4 in arbitrary direction, and the small pieces of nonwoven fabric had an overall length of about 25 mm in a plan view.

Performance Evaluation

For each of the absorbent members produced in Examples and Comparative Examples, compression workload in a dry state (d-WC), compression workload in a wet state (w-WC), compression recovery rate in a dry state (d-RC), compression recovery rate in a wet state (w-RC), torsion moment in a dry state, and torsion moment in a wet state were measured according to the methods described above. The results are shown in Table 1 given below.

The torsion moment was measured on an absorbent member with the one side thereof covered with a top sheet, which was regarded as an absorbent article. The specifications of the top sheets used in the measurement were as follows.

- Other than Comparative Example 1: an air-through non-woven fabric formed of fibers made of polyethylene resin and polyethylene terephthalate resin and having a basis weight of 74 $g/m^2$ and a thickness of 1.4 mm.
- Comparative Example 1: the top sheet (basis weight: 27 $g/m^2$) of a sanitary napkin (product name: Tanom Pew Slim 23 cm) available from Unicharm Corporation

[Table 1]

| | | Example | | | | Comparative Example | |
|---|---|---|---|---|---|---|---|
| | | 1 | 2 | 3 | 4 | 1 | 2 |
| Fiber clusters | | Yes | Yes | Yes | Yes | No | Yes |
| Method of bonding fiber clusters | | Entanglement | Entanglement | Entanglement | Entanglement | - | Thermal fusion |
| Amount of fiber cluster (mass%) | | 25 | 50 | 50 | 75 | | 50 |
| Dimensions of fiber cluster (mm) | | $0.8 \times 3.9$ | $0.8 \times 3.9$ | $0.8 \times 3.9$ | $0.8 \times 3.9$ | - | 25* |
| Basis weight of fiber cluster (g/m$^2$) | | 50 | 100 | 100 | 150 | 116*2 | 100 |
| Basis weight of absorbent fiber (g/m$^2$) | | 150 | 100 | 100 | 50 | 116 | 100 |
| Basis weight of Absorbent polymer (g/m$^2$) | | 0 | 0 | 50 | 0 | 35 | 0 |
| Thickness of absorbent member (mm) | | 2.8 | 2.8 | 2.8 | 2.8 | 2.8 | 2.8 |
| Absorbent member in dry state | d-WC (mv·cm/cm$^2$) | 32.84 | 36.76 | 35.78 | 39.22 | 19.12 | 24.02 |
| | d-RC (%) | 43 | 45 | 47 | 50 | 40 | 52 |
| Absorbent member in wet state | w-WC (mv·cm/cm$^2$ ) | 29.41 | 32.35 | 31.37 | 36.27 | 15.20 | 19.61 |
| | w-RC (%) | 40 | 42 | 43 | 48 | 25 | 47 |
| Torsion moment (mv·m) m) | Stack including absorbent member in dry state | 0.52 | 0.44 | 0.48 | 0.42 | 0.55 | 1.25 |
| | Stack including absorbent member in wet state | 0.64 | 0.55 | 0.60 | 0.50 | 0.84 | 1.45 |

*1: Overall length of fiber cluster
*2: The basis weight of synthetic fibers contained in the absorbent member is shown because the absorbent member did not contain fiber clusters.

As shown in Table 1, the absorbent members produced in Examples contained fiber clusters that were "fiber wads having a definite shape" each defined by two base surfaces and a body surface intersecting the two base surfaces. For this reason, in both the dry state and the wet state, the compression workload was larger and the torsion moment of the absorbent article was smaller than those of Comparative Examples 1 and 2, which did not contain fiber clusters having a definite shape. As can be seen from the above, the absorbent member containing a fiber cluster having a definite shape as described above is flexible, has excellent cushioning properties and excellent compression recovery, and can deform in good response to an external force, and thus the absorbent member can provide improved wearing comfort and a better fit when it is used in an absorbent article.

Industrial Applicability

The absorbent member according to the present invention has excellent cushioning properties and excellent compression recovery, is capable of flexibly deforming in response to an external force, and can provide improved wearing comfort and a better fit when it is used in an absorbent article.

Also, the absorbent article according to the present invention includes the high quality absorbent member, and thus can provide excellent wearing comfort and an excellent fit.

**Claims**

1. An absorbent member comprising:

   a plurality of fiber clusters containing synthetic fibers; and
   a plurality of absorbent fibers,
   wherein the fiber clusters are entangled with each other, or the fiber clusters and the absorbent fibers are entangled,
   each of the fiber clusters includes two opposing base surfaces and a body surface that connects the two base surfaces, and
   the number of fiber end portions per unit area in the body surface is greater than the number of fiber end portions per unit area in each of the base surfaces.

2. The absorbent member according to claim 1,
   wherein a total area of the two base surfaces is larger than a total area of the body surface.

3. The absorbent member according to claim 1 or 2,
   wherein each of the fiber clusters includes an extended fiber bundle portion extending outward from the body surface and containing a plurality of fibers.

4. The absorbent member according to claim 3,
   wherein the extended fiber bundle portion includes a thermally fused portion in which a plurality of fibers are thermally fused to each other.

5. The absorbent member according to claim 3 or 4,
   wherein each of the fiber clusters includes a main body portion defined by the base surfaces and the body surface, and an extension length of the extended fiber bundle portion from the main body portion is 0.2 mm or more and 7 mm or less.

6. The absorbent member according to any one of claims 1 to 5,
   wherein an outer shape of each of the fiber clusters is a rectangular parallelepiped shape or a disc shape.

7. The absorbent member according to any one of claims 1 to 6,
   wherein each of the base surfaces has a rectangular shape in a plan view, and a short side of the rectangular shape is smaller than or equal to a thickness of the absorbent member.

8. The absorbent member according to claim 7,
   wherein a ratio of a length of the short side of each of the base surfaces to the thickness of the absorbent member, former / latter, is 0.08 or more and 0.5 or less.

9. The absorbent member according to claim 7 or 8,
   wherein the length of the short side of each of the base surfaces is 0.3 mm or more and 10 mm or less.

10. The absorbent member according to any one of claims 7 to 9,
    wherein the length of a long side of each of the base surfaces is 0.3 mm or more and 30 mm or less.

11. The absorbent member according to any one of claims 1 to 10,
    wherein, in projected views in two directions of the absorbent member that are orthogonal to each other, there are overlapping portions of the plurality of fiber clusters in an arbitral unit region of 10 mm square.

12. The absorbent member according to any one of claims 1 to 11,
    wherein each of the fiber clusters contains a plurality of thermoplastic fibers as the synthetic fibers, and has a three-dimensional structure in which the plurality of thermoplastic fibers are thermally fused to each other.

13. The absorbent member according to any one of claims 1 to 12,
    wherein a mass ratio of the fiber clusters and the absorbent fibers included, former / latter, is 20/80 to 80/20.

14. The absorbent member according to any one of claims 1 to 13,
    wherein, in the absorbent member, some fiber clusters are bonded to other fiber clusters or the absorbent fibers by means of entanglement, and some fiber clusters are capable of being entangled with other fiber clusters or the absorbent fibers.

15. The absorbent member according to claim 14,
    wherein the total number of the fiber clusters bonded through entanglement and the fiber clusters capable of being entangled accounts for 50% or more relative to the total number of the fiber clusters in the absorbent member.

16. The absorbent member according to any one of claims 1 to 15,
    wherein the number of the fiber clusters having bonding portions to other fiber clusters or the absorbent fibers through entanglement of fibers accounts for 70% or more of the total number of the fiber clusters having bonding portions to other fiber clusters or the absorbent fibers.

17. The absorbent member according to any one of claims 1 to 16,
    wherein the fiber clusters are derived from a nonwoven fabric.

18. The absorbent member according to any one of claims 1 to 17,
    wherein a ratio of the number $N_1$ of the fiber end portions per unit area in the base surfaces to the number $N_2$ of the fiber end portions per unit area in the body surface, $N_1/N_2$, is 0 or more and 0.90 or less.

19. The absorbent member according to any one of claims 1 to 18,
    wherein the number of fiber end portions per unit area in the base surfaces is 0 fiber end portions/mm$^2$ or more and 8 fiber end portions/mm$^2$ or less.

20. The absorbent member according to any one of claims 1 to 19,
    wherein the number of fiber end portions per unit area in the body surface is 5 fiber end portions/mm$^2$ or more and 50 fiber end portions/mm$^2$ or less.

21. An absorbent member comprising:

    a plurality of fiber clusters containing synthetic fibers; and
    a plurality of absorbent fibers,
    wherein the fiber clusters are entangled with each other, or the fiber clusters and the absorbent fibers are entangled,
    each of the fiber clusters includes two opposing base surfaces and a body surface that connects the two base surfaces, and
    each of the fiber clusters includes an extended fiber bundle portion extending outward from the body surface and containing a plurality of fibers.

22. The absorbent member according to claim 21,

wherein a total area of the two base surfaces is larger than a total area of the body surface.

23. An absorbent article comprising the absorbent member according to any one of claims 1 to 22.

24. The absorbent article according to claim 23,
   wherein the absorbent article in a dry state has a torsion moment of 0.20 mN·m or more and 1.30 mN·m or less.

25. The absorbent article according to claim 23 or 24,
   wherein the absorbent article in a wet state has a torsion moment of 0.20 mN·m or more and 1.20 mN·m or less.

Fig. 1

Fig. 2

Fig. 3(a)

Fig. 3(b)

# Fig. 4

Fig. 5(a)

Fig. 5(b)

Fig. 6

Fig. 7

## INTERNATIONAL SEARCH REPORT

| International application No. |
|---|
| PCT/JP2018/036768 |

**A. CLASSIFICATION OF SUBJECT MATTER**
Int. Cl. A61F13/53(2006.01)i, D04H1/46(2012.01)i

According to International Patent Classification (IPC) or to both national classification and IPC

**B. FIELDS SEARCHED**

Minimum documentation searched (classification system followed by classification symbols)
Int. Cl. A61F13/15-13/84, D04H1/00-18/04

Documentation searched other than minimum documentation to the extent that such documents are included in the fields searched
Published examined utility model applications of Japan      1922-1996
Published unexamined utility model applications of Japan    1971-2018
Registered utility model specifications of Japan            1996-2018
Published registered utility model applications of Japan    1994-2018

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2002-301105 A (UNI-CHARM CORP.) 15 October 2002 & US 2002/0147434 A1 & US 8182623 B2 & EP 1247509 B1 & AT 406149 T & KR 2002-0079471 A & SG 105542 A & TW 546134 B | 1-25 |
| A | JP 2001-212173 A (UNI-CHARM CORP.) 07 August 2001 & US 6533771 B2 & EP 1121915 B1 & EP 1649844 A2 & AU 776490 B2 & BR 01100319 B1 & CA 2332741 C & CN 1160032 C & CN 1313073 A & DE 60122399 T2 & ID 29119 A & KR 10-0695701 B1 & MY 126106 A & SG 101952 A1 & TW 497412 U | 1-25 |
| A | JP 7-24003 A (KAO CORP.) 27 January 1995 (Family: none) | 1-25 |

☒ Further documents are listed in the continuation of Box C.        ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 29.11.2018 | 11.12.2018 |

| Name and mailing address of the ISA/ Japan Patent Office 3-4-3, Kasumigaseki, Chiyoda-ku, Tokyo 100-8915, Japan | Authorized officer |
|---|---|
| | Telephone No. |

Form PCT/ISA/210 (second sheet) (January 2015)

**INTERNATIONAL SEARCH REPORT**

| International application No. |
| --- |
| PCT/JP2018/036768 |

C (Continuation).  DOCUMENTS CONSIDERED TO BE RELEVANT

| Category* | Citation of document, with indication, where appropriate, of the relevant passages | Relevant to claim No. |
| --- | --- | --- |
| A | JP 2003-39585 A (UNI-CHARM CORP.) 13 February 2003 (Family: none) | 1-25 |
| A | JP 2005-237952 A (KAO CORP.) 08 September 2005 (Family: none) | 1-25 |
| A | JP 2017-119024 A (UNI-CHARM CORP.) 06 July 2017 & WO 2017/115523 A1 & EP 3381427 A1 & AU 2016381713 A1 & CA 3009936 A1 & CN 108472181 A & KR 10-2018-0098270 A & TW 201729768 A | 1-25 |

Form PCT/ISA/210 (continuation of second sheet) (January 2015)

## REFERENCES CITED IN THE DESCRIPTION

**Patent documents cited in the description**

- US 20150366726 A
- US 20100174259 A
- US 4813948 A

**Non-patent literature cited in the description**

- **SUEO KAWABATA.** Standardization and Analysis of Texture Evaluation. Kato Tech Co., Ltd, 10 July 1980